# EUROPEAN PATENT APPLICATION

(11) **EP 4 725 490 A1**
(43) Date of publication of application: **15.04.2026**
(21) Application number: 24818729.6
(22) Date of filing: 06.06.2024
(51) Int. Cl.: A61K 31/519, A61K 31/395, A61K 9/14, A61K 47/38, A61K 47/32, A61P 35/00

(54) **EED INHIBITOR SOLID DISPERSION, ORAL FORMULATION COMPRISING SAME, AND PREPARATION METHOD THEREFOR**

(30) Priority: 06.06.2023 WO PCT/CN2023/098642
(71) Applicant: Ascentage Pharma (Suzhou) Co., Ltd., Suzhou, Jiangsu 215127 (CN); Ascentage Pharma Group Corp Limited, Hong Kong (CN)
(72) Inventor: LIN, Yanqiong, Suzhou, Jiangsu 215127 (CN); GUO, Hongtao, Suzhou, Jiangsu 215127 (CN); XU, Feng, Suzhou, Jiangsu 215127 (CN)
(74) Representative: AWA Denmark A/S
(86) International application number: PCT/CN2024/097747
(87) International publication number: WO 2024/251197

(57) **Abstract**

The present disclosure relates to the field of pharmaceutical formulations, and particularly relates to a solid dispersion having good dissolution and good bioavailability and comprising an embryonic ectoderm development (EED) inhibitor and a preparation method therefor, a solid dispersion composition comprising the solid dispersion and an excipient, and an oral formulation comprising the solid dispersion or the solid dispersion composition, as well as a preparation method therefor, and use of the solid dispersion, the solid dispersion composition, or the oral formulation in treating and/or preventing an EED-mediated disease.

## Description

### TECHNICAL FIELD

The present disclosure pertains to the field of pharmaceutical formulations, and particularly relates to a solid dispersion comprising an embryonic ectoderm development (EED) protein inhibitor, a solid dispersion composition comprising the solid dispersion and an excipient, and an oral formulation comprising the solid dispersion or the solid dispersion composition that have good dissolution and good bioavailability, as well as a preparation method therefor, and use of the solid dispersion, the solid dispersion composition, or the oral formulation in treating and/or preventing an EED-mediated disease.

### BACKGROUND

Polycomb group (PcG) proteins are a group of transcriptional repressors that regulate target genes through chromatin modification. They not only control the normal development pattern of individuals, but also are closely related to cell proliferation, differentiation, and tumorigenesis. PcG proteins can be divided into two major classes: PRC1 (polycomb repressive complex 1, with E3 ubiquitin ligase activity) and PRC2 (polycomb repressive complex 2, with methyltransferase activity).

PRC2, as a representative member of PcG, is a multi-subunit complex that maintains the chromatin inhibitory state by silencing the expression of specific genes. It plays a key role in development, tissue differentiation, and regeneration, and its core subunits include enhancer of zeste homolog 2 (EZH2), EED, suppressor of zeste 12 (SUZ12), and retinoblastoma repressor-associated protein 46/48 (RbAp46/48). Deregulation of PRC2 has been found in a number of human cancers. For example, EZH2 is highly expressed in a variety of human cancers and promotes carcinogenesis and malignant transformation. EZH2 mutations occur in up to 25% of diffuse large B-cell lymphoma (DLBCL) and follicular lymphoma (FL), and are associated with poor patient prognosis.

Targeted inhibition of the methyltransferase activity of EZH2 has been shown to be a successful cancer treatment strategy. However, a secondary mutation of EZH2 can result in acquired resistance, and its homologous EZH1 also has methyltransferase activity, both of which can lead to limited activity of EZH2 inhibitors. Since EED can activate the methyltransferase activity of EZH2, allosteric targeted inhibition of EED is also an effective anti-cancer method, and can produce a stronger anti-tumor effect by overcoming the resistance to EZH2 inhibitors and inhibiting both EZH2 and EZH1. Previous study results have indeed shown that EED inhibitors have great therapeutic potential in hematological tumors, solid tumors, and non-tumor indications.

To date, EED inhibitors that have entered clinical research include MAK683 developed by Novartis and FTX-6058 developed by Fulcrum Therapeutics. In addition, Ascentage Pharma is developing a novel, potent, and selective small-molecule EED inhibitor, which exhibits high EED-binding affinity. By regulating tumor epigenetics and the tumor microenvironment, this inhibitor is expected to overcome tumor drug resistance and achieve complete and durable tumor regression. On June 29, 2022 and November 10, 2022 respectively, this compound obtained clinical trial approval from the U.S. FDA and the Center for Drug Evaluation (CDE) of the National Medical Products Administration (NMPA) of China, for conducting a Phase I clinical trial in the treatment of advanced solid tumors or hematological malignancies. It is also the first domestically developed EED inhibitor to enter the clinical stage in China.

A compound with the chemical name of 12-(((5-fluoro-2,3-dihydrobenzofuran-4-yl)methyl)amino)-4-isopropyl-7-(trifluoromethyl)-4,5-dihydro-3H-2,4,8,11,12a-pentaazabenzo[4,5]cycloocta[1,2,3-cd]inden-3-one is a member of a group of imidazopyrimidine EED inhibitors disclosed in WO2021/011713 (CN114127073A), and has a structural formula shown as follows: 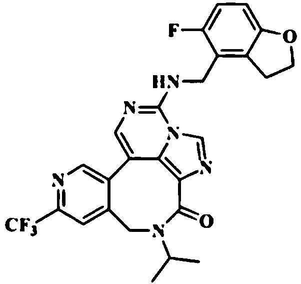 (hereinafter referred to as "compound A")

Preclinical data have demonstrated that compound A has *in vitro* anti-tumor cell proliferation activity in multiple tumor cell lines, and anti-tumor activity in PDX/CDX models of EZH2-mutant B-cell non-Hodgkin lymphoma, INI1-negative malignant rhabdoid tumor, BAP1-mutant mesothelioma, and prostate cancer.

In view of the great therapeutic potential of EED inhibitors in the field of tumor treatment, the early development of EED inhibitor pharmaceutical formulations that are easy to prepare and convenient to use and have good formulation properties can benefit more tumor patients. Meanwhile, in view of the safety problem, i.e., toxic and side effects, which has long been concerned in the field of tumor therapeutic drugs, there is a great need in practice for anti-cancer drugs with enhanced bioavailability that can exert therapeutic efficacy at a relatively low dose.

The starting material of compound A is an anhydrous crystal powder with slight hygroscopicity, which is almost insoluble in water. The solubility of compound A in an aqueous buffer with pH of 1.2 to 7.4 is 0.39-0.08 µg/mL (37 °C/24 h), and the fluidity of compound A is poor. For such drugs with poor water solubility, the dissolution of the drugs in gastrointestinal fluids is the rate-limiting step of their bioavailability, and poor water solubility will necessarily lead to low dissolution and thus lower bioavailability. Therefore, if the bioavailability of the drug is to be improved, the dosage form, formula composition, and process need to be specially designed to change the dispersion state of the drug and promote its dissolution in the gastrointestinal tract.

In order to meet the above needs and solve the above problems, the inventors have developed a stable EED inhibitor solid dispersion capable of providing good dissolution and good bioavailability through extensive research and screening, and prepared the solid dispersion into an oral formulation with good formulation properties through formula design, thereby achieving the purpose of the present disclosure.

### BRIEF SUMMARY

In one aspect, the present disclosure provides a stable solid dispersion having good dissolution and bioavailability, which comprises an EED inhibitor (especially compound A) and a carrier substance.

In another aspect, the present disclosure provides a stable solid dispersion composition having good dissolution and bioavailability, which comprises the solid dispersion of the present disclosure and one or more pharmaceutically acceptable excipients.

In another aspect, the present disclosure provides an oral formulation (e.g., tablet) having good dissolution and bioavailability, which comprises the solid dispersion or the solid dispersion composition of the present disclosure, wherein the EED inhibitor is in the solid dispersion.

In another aspect, the present disclosure provides a method for preparing the solid dispersion of the present disclosure.

In another aspect, the present disclosure provides a method for preparing the oral formulation of the present disclosure.

In another aspect, the present disclosure provides a method for treating or preventing a disease (e.g., cancer or tumor) where inhibition of EED provides a benefit, which comprises administering a therapeutically effective amount of the solid dispersion, the solid dispersion composition, or the oral formulation of the present disclosure to a subject in need.

In another aspect, the present disclosure provides use of the solid dispersion, the solid dispersion composition, or the oral formulation of the present disclosure in treating or preventing a disease (e.g., cancer or tumor) where inhibition of EED provides a benefit.

In another aspect, the present disclosure provides use of the solid dispersion, the solid dispersion composition, or the oral formulation of the present disclosure in preparing a medicament for treating or preventing a disease (e.g., cancer or tumor) where inhibition of EED provides a benefit.

### Detailed Description of the Invention

### Definitions

The term "about" as used herein shall allow for a ±10% fluctuation of a numerical value used in conjunction therewith. For ratios, the term "about" is used to define every number of a given ratio. For example, a ratio of about 1:1 refers to a ratio of 0.9-1.1:0.9-1.1.

"Solid dispersion" refers to a highly dispersed system formed by uniformly dispersing a drug in an inactive auxiliary material or carrier in a highly dispersed state such as a molecular, colloidal, amorphous, or microcrystalline state. Characteristics of solid dispersions include the following: The solid dispersions can delay drug hydrolysis and oxidation, can mask unpleasant odors and irritation, can solidify liquid drugs, and can also accelerate or delay drug dissolution to achieve the purpose of immediate release or sustained release. However, the highly dispersed state also brings problems to solid dispersions, including poor physical stability and susceptibility to aging after long-term storage.

Different types of solid dispersions exist due to different carrier materials, preparation methods, composition ratios, etc., including, but not limited to, simple eutectic mixtures, solid solutions, and co-precipitates. There are various methods for preparing the solid dispersion, including a melt method, a solvent method, a solvent-melt method, a solvent-spray drying method, a grinding method, and the like. The means for verifying and analyzing the presence of the drug and identifying the formation of the solid dispersion include thermal analysis (including differential thermal analysis (DTA) or differential scanning calorimetry (DSC)), X-ray powder diffraction (XRPD), infrared spectroscopy, polarized light microscopy (PLM), and the like.

However, in view of the diversity of the types and properties of the carrier substances, the diversity of the preparation methods, and the frequent incompatibility between the carrier substances/preparation methods and specific drug molecules, the advantages of the solid dispersions described above are often not achieved. That is, a solid dispersion that can bring good formulation properties is often not obtained only based on the molecular structure and physicochemical properties of the specific drug and conventional technical means for solid dispersions.

The "solid dispersion carrier" is a material in which the drug in the solid dispersion is dispersed in a highly dispersed state such as a molecular, colloidal, amorphous, or microcrystalline state. The properties of the carrier material have a great influence on the properties of the solid dispersion. The carrier material should have the basic properties including non-toxicity, non-carcinogenicity, no impact on drug stability, no chemical reaction with the drug, and no interference with the efficacy or content monitoring of the drug, and the like.

Commonly used solid dispersion carrier materials can be divided into: water-soluble carrier materials including polyethylene glycol (PEG), polyvinylpyrrolidone (PVP), surfactants, organic acids, sugars (alcohols), etc.; poorly soluble carrier materials including celluloses, polyacrylic acid resins, lipids, etc.; enteric carrier materials including enteric celluloses, enteric polyacrylic acid resins, etc. It is generally believed that a water-soluble carrier can increase the solubility and dissolution rate of a poorly soluble drug and is helpful to improve the bioavailability of the drug; a poorly soluble carrier can delay or control the drug release; an enteric carrier can control the drug release in the small intestine.

Among them, polyethylene glycol carriers are one of the most commonly used water-soluble carriers, and are characterized by low toxicity, low melting point, and good water solubility. After being prepared into a solid dispersion, polyethylene glycol carriers can disperse drug molecules in a molecular state, thereby accelerating the dissolution rate of the drug. PEG with a molecular weight of 1000-20000 is generally selected as the solid dispersion carrier, and PEG4000 and PEG6000 are most commonly used.

Povidone carriers are amorphous high-molecular polymers with thermal stability, readily soluble in water and polar organic solvents such as ethanol, and exhibit relatively strong crystal inhibition effects on a variety of drugs.

Polyacrylic acid resin carriers are a generic term for copolymers of acrylic acid, methacrylic acid, and derivatives thereof. The most commonly used products, under the trade name Eudragit, comprise methacrylic acid copolymers and methacrylate copolymers and are classified into different models according to different compositions, ratios, and degrees of polymerization. Eudragit E is a copolymer of dimethylaminoethyl methacrylate and other neutral methacrylates, and Eudragit L and Eudragit S are copolymers of methacrylic acid and acrylic esters in different ratios, including a methacrylic acid-ethyl acrylate (1:1) copolymer, a methacrylic acid-methyl methacrylate (1:1) copolymer, and a methacrylic acid-methyl methacrylate (1:2) copolymer. Specific examples include, but are not limited to, Eudragit E100, Eudragit EPO, Eudragit L100-55, Eudragit L30D-55, Eudragit L100, Eudragit S100, etc.

Examples of the cellulose carrier include ethylcellulose, hydroxypropyl cellulose, and hydroxypropyl methylcellulose, and further include enteric cellulose, such as cellulose acetate phthalate, hydroxypropyl methylcellulose phthalate, hydroxypropyl methylcellulose acetate succinate, and the like.

The term "formulation" or "pharmaceutical composition" as used herein refers to a composition which is suitably administered to animals, preferably mammals (including humans) and comprises at least one active ingredient and at least one inactive ingredient, such as a pharmaceutically acceptable excipient. The formulation of the present disclosure may be any formulation suitable in the art, such as a tablet, a capsule, or a liquid formulation.

The term "pharmaceutically acceptable excipient" as used herein refers to an ingredient in a pharmaceutical formulation, other than an active ingredient, which is non-toxic to an individual. Examples of the pharmaceutically acceptable carrier include, but are not limited to, binders, disintegrants, lubricants, solvents, dispersion media, buffers, excipients, antioxidants, preservatives, flavoring agents, or the like.

The term "EED inhibitor" as used herein refers to a bioactive agent that can reduce the signaling activity of EED by interacting with the EED protein directly or indirectly, in particular to a group of imidazopyrimidine EED inhibitors having formula (I) described in CN114127073A, more particularly the EED inhibitor compounds having formula (III) therein, and most preferably the compound of Example 73, or a pharmaceutically acceptable salt or solvate thereof. The entire contents of CN114127073A are incorporated herein by reference.

The term "EED-mediated disease" or "disease where inhibition of EED provides a benefit" as used herein refers to a disease or disorder in which EED is involved in the occurrence of the disease or disorder, and in the onset, severity, or progression of one or more of its disease symptoms or markers; or a disease or disorder in which inhibition of EED will reduce the incidence of the disease and reduce or eliminate disease symptoms. Such diseases or disorders include, but are not limited to, cancer and proliferative diseases, inflammatory diseases, sepsis, autoimmune diseases, and viral infections, among which cancer and proliferative diseases are the most significant. Those of ordinary skill in the art are readily able to determine whether a compound treats a disease or condition mediated by EED for any particular cell type, for example, by assays that can be conveniently used to assess the activity of the specific compound. See, e.g., Yue and Turkson, Expert Opinion Invest Drugs, 18:45-56 (2009).

The term "patient", "subject", "participant", or "individual" as used herein refers to mammalian and non-mammalian individuals in need of the EED inhibitor of the present disclosure. Examples include, but are not limited to, primates (e.g., humans and non-human primates such as monkeys), horses, cows, sheep, cats, dogs, rabbits, and rodents (e.g., mice and rats), without limitation to a specific age or sex. In some embodiments, the term refers to a human, including a child, a teenager, or an adult.

The term "treat", "treating", or "treatment" as used herein refers to administration of the EED inhibitor solid dispersion or the pharmaceutical composition comprising the same of the present disclosure to an individual having the relevant disease or a symptom thereof, or to an individual predisposed to the relevant disease, for the purpose of curing, alleviating, or ameliorating the disease or a symptom thereof, or preventing the individual from developing the disease. In a specific embodiment of the present disclosure, the disease is an EED-mediated disease as defined above, particularly a tumor or cancer.

The term "prevent", "preventing", or "prevention" as used herein refers to administration of the EED inhibitor solid dispersion or the pharmaceutical composition comprising the same of the present disclosure to a subject, e.g., a mammal, such as a human, suspected to suffer from or susceptible to the EED-mediated disease, in particular cancer or tumor, as defined herein, to delay the onset of the disease or reduce the risk of suffering from the disease. The term "prevent", "preventing", or "prevention" includes the use of the solid dispersion or the pharmaceutical composition of the present disclosure prior to the diagnosis or identification of any clinical and/or pathological symptom.

The term "effective amount" as used herein refers to the amount of a pharmaceutically active agent that, when administered to a specific patient or patient population, alone or in combination with additional therapeutic agents, is sufficient to prevent the occurrence of one or more symptoms of the disease or disorder being treated or alleviate the symptoms of the disease to some extent. The effective amount of the EED inhibitor in the solid dispersion or the pharmaceutical composition of the present disclosure is generally about 1 µg/kg to 100 mg/kg per day, for example, a daily dosage of about 1 mg/kg to about 10 mg/kg. In some cases, it is possible that the effective amount of the EED inhibitor of the present disclosure is above the upper limit of the dosage range described above or below the lower limit of the dose range described above. Those skilled in the relevant art can determine the effective amount of the EED inhibitor pharmaceutical composition of the present disclosure by conventional methods (e.g., modeling, dose-escalation studies, or clinical trials) in combination with conventional influencing factors (e.g., route of administration, pharmacokinetics of the compound, severity and course of the disease, medical history of the individual, health status of the individual, and degree of the individual's response to the drug).

The term "AUCₗₐₛₜ" as used herein refers to the area under the plasma concentration-time curve from the time of administration to the last measurable time point, expressed in ng·h·mL⁻¹.

The term "AUC_{INF}" as used herein refers to the area under the plasma concentration-time curve calculated by extrapolating the measured values from the time of administration (0 h) to infinity, expressed in ng·h·mL⁻¹.

The term "Cₘₐₓ" as used herein refers to the highest concentration of a drug in plasma after administration, expressed in ng/mL.

The term "Tₘₐₓ" as used herein refers to the time at which Cₘₐₓ occurs after administration, expressed in hour (h).

The term "T_{1/2}" as used herein refers to the time required for half of the plasma drug concentration to be eliminated after the drug distribution in the body reaches an equilibrium, expressed in hour (h).

Typical acceptable criteria for the term "stable" as used herein to describe the solid dispersion or oral formulation of the present disclosure are as follows: As determined by the HPLC method described herein, a total impurity content is no more than about 1%, preferably no more than about 0.5%, e.g., the total impurity content may be 0.01%, 0.05%, 0.1%, 0.2%, 0.3%, 0.4%, 0.5%, 0.6%, 0.7%, 0.8%, 0.9%, or 1.0%; and/or the physical form of the active ingredient in the solid dispersion/oral formulation remains amorphous with no significant crystal presence or aging as detected by the XRPD, DTA, DSC, or PLM method.

The expression "by weight of the oral formulation" as used herein when describing the content of each ingredient refers to the content of the active ingredient and other types of excipients calculated based on the weight of the tablet core excluding a coating agent.

The reference to any EED inhibitor appearing herein is intended to include free compounds, and pharmaceutically acceptable salts, hydrates, or solvates thereof.

The term "pharmaceutically acceptable salt" as used herein refers to a salt or a zwitterionic form of the EED inhibitor compound involved in the present disclosure. The pharmaceutically acceptable salt of the EED inhibitor involved in the present disclosure may be an acid addition salt formed with a pharmaceutically acceptable acid. Examples include inorganic acids such as nitric acid, boric acid, hydrochloric acid, hydrobromic acid, sulfuric acid, and phosphoric acid, and organic acids such as oxalic acid, maleic acid, succinic acid, and citric acid. Non-limiting examples of salts of the EED inhibitor involved in the present disclosure include, but are not limited to, hydrochloride, hydrobromide, hydroiodide, sulfate, bisulfate, 2-hydroxyethanesulfonate, phosphate, hydrogen phosphate, acetate, adipate, alginate, aspartate, benzoate, bisulfate, butyrate, camphorate, camphorsulfonate, digluconate, glycerophosphate, hemisulfate, heptanoate, hexanoate, formate, succinate, fumarate, maleate, ascorbate, isethionate, salicylate, methanesulfonate, mesitylenesulfonate, naphthylenesulfonate, nicotinate, 2-naphthalenesulfonate, oxalate, pamoate, pectinate, persulfate, 3-phenylpropionate, picrate, pivalate, propionate, trichloroacetate, trifluoroacetate, phosphate, glutamate, bicarbonate, p-toluenesulfonate, undecanoate, lactate, citrate, tartrate, gluconate, methanesulfonate, ethanedisulfonate, benzenesulfonate, and p-toluenesulfonate. In addition, available amino groups present in the EED inhibitor compounds involved in the present disclosure can be quaternized with methyl, ethyl, propyl, and butyl chlorides, bromides, and iodides; dimethyl, diethyl, dibutyl, and dipentyl sulfates; decyl, lauryl, myristyl, and sterol chlorides, bromides, and iodides; and benzyl and phenethyl bromides.

The term "solvate" as used herein refers to a combination, physical association, and/or fusion of the EED inhibitor compound involved in the present disclosure with a solvent molecule, e.g., a disolvate, monosolvate, or hemisolvate, wherein a ratio of the solvent molecule to the compound of the present disclosure is about 2:1, about 1:1, or about 1:2, respectively. Solvates can generally serve as pharmacological equivalents. The EED inhibitor compounds involved in the present disclosure may exist in solvated forms with pharmaceutically acceptable solvents such as water, methanol, and ethanol. One type of solvate is a hydrate. "Hydrate" refers to a specific subgroup of solvates, wherein the solvent molecule is water.

The EED inhibitor involved in the present disclosure encompasses any compounds of the present disclosure that are isotopically labeled (i.e., radiolabeled) by substituting one or more atoms with atoms having a different atomic mass or mass number. Examples of isotopes that can be incorporated into the disclosed compounds include isotopes of hydrogen, carbon, nitrogen, oxygen, phosphorus, fluorine, and chlorine, such as ²H (or deuterium (D)), ³H, ¹¹C, ¹³C, ¹⁴C, ¹⁵N, ¹⁸O, ¹⁷O, ³¹P, ³²P, ³⁵S, ¹⁸F, and ³⁸Cl, respectively, e.g., ³H, ¹¹C, and ¹⁴C. In one embodiment, substantially all atoms at a certain position of the EED inhibitor involved in the present disclosure are substituted with atoms having a different atomic mass or mass number. In another embodiment, substantially all atoms at a certain position of the EED inhibitor involved in the present disclosure are substituted with deuterium atoms; for example, all hydrogen atoms of the -CH₃ group are substituted with deuterium atoms to give a -CD₃ group. In another embodiment, some atoms at a certain position of the EED inhibitor involved in the present disclosure are substituted with atoms having a different atomic mass or mass number. In another embodiment, none of the atoms of the EED inhibitor involved in the present disclosure is substituted with atoms having a different atomic mass or mass number. Isotopically-labeled EED inhibitors involved in the present disclosure can be prepared by methods known in the art.

The EED inhibitor involved in the present disclosure may contain one or more asymmetric centers and may thus give rise to enantiomers, diastereomers, and other stereoisomeric forms. The present disclosure encompasses use of all such possible forms, as well as their racemic and resolved forms and mixtures thereof.

Technical and scientific terms as used herein that are not specifically defined have the same meaning as commonly understood by those skilled in the art to which the present disclosure relates.

### Detailed Description of the Invention

In **a first aspect,** the present disclosure provides a solid dispersion comprising an EED inhibitor or a pharmaceutically acceptable salt or solvate thereof and a polymeric carrier.

In one embodiment, the present disclosure provides the solid dispersion described above, wherein the EED inhibitor is the EED inhibitor described in CN114127073A, which is incorporated herein in its entirety. Specifically, the EED inhibitor involved in the present disclosure has the following formula: 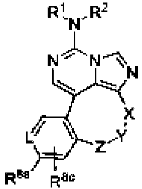 wherein:
R¹ is aralkyl;
R² is selected from H and C₁-C₄ alkyl;
X is selected from -C(R^{5a})(R^{5b})-, -C(=O)-, and -S(=O)₂-; R^{5a} and R^{5b} are independently selected from H and C₁-C₄ alkyl;
Y is selected from -C(R^{6a})(R^{6b})-, -S-, -O-, and -N(R⁷)-;
Z is -C(R^{6c})(R^{6d})ₘ-;
R^{6a} and R^{6b} are independently selected from H and C₁-C₄ alkyl;
R^{6c} and R^{6d} are each independently selected from H and C₁-C₄ alkyl; m is 0, 1, or 2;
R⁷ is selected from C₁-C₆ alkyl, C₁-C₆ haloalkyl, and optionally substituted C₃-C₈ cycloalkyl;
L is selected from -C(R^{8b})= and -N=;
R^{8a} is selected from -CF₃, -CH₃, -CHF₂, -CD₃, and cyclopropyl; and
R^{8b} and R^{8c} are hydrogen;
or the EED inhibitor involved in the present disclosure is a pharmaceutically acceptable salt or solvate thereof.

In one embodiment, the present disclosure provides the solid dispersion described above, wherein the EED inhibitor is selected from:

| | |
|---|---|
| 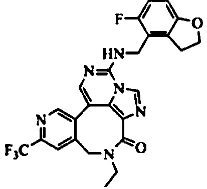 | 4-Ethyl-12-(((5-fluoro-2,3-dihydrobenzofuran-4-yl)methyl)amino)-7-(trifluoromethyl)-4,5-dihydro-3H-2,4,8,11,12a-pentaazabenzo[4,5]cycloocta[1,2,3-cd]inden-3-one |
| 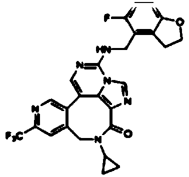 | 4-Cyclopropyl-12-(((5-fluoro-2,3-dihydrobenzofuran-4-yl)methyl)amino)-7-(trifluoromethyl)-4,5-dihydro-3H-2,4,8,11,12a-pentaazabenzo[4,5]cycloocta[1,2,3-cd]inden-3-one |
| 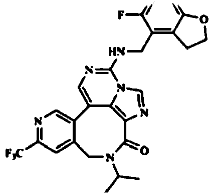 | 12-(((5-Fluoro-2,3-dihydrobenzofuran-4-yl)methyl)amino)-4-isopropyl-7-(trifluoromethyl)-4,5-dihydro-3H-2,4,8,11,12a-pentaazabenzo[4,5]cycloocta[1,2,3-cd]inden-3-one |

or a pharmaceutically acceptable salt or solvate thereof.

In one embodiment, the present disclosure provides the solid dispersion described above, wherein the EED inhibitor is compound A 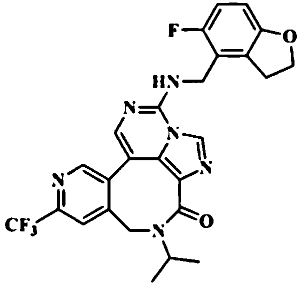 or a pharmaceutically acceptable salt or solvate thereof.

In one embodiment, the present disclosure provides the solid dispersion described above, wherein the EED inhibitor, e.g., compound A, can form a salt with an acid to give a pharmaceutically acceptable salt. The acid is well known to those skilled in the art or can be conventionally determined, for example, but is not limited to, hydrochloric acid, methanesulfonic acid, fumaric acid, trifluoroacetic acid or phosphoric acid, sulfuric acid, 2-hydroxyethanesulfonic acid, acetic acid, benzoic acid, succinic acid, maleic acid, oxalic acid, p-toluenesulfonic acid, citric acid, tartaric acid, and benzenesulfonic acid.

In one embodiment, the present disclosure provides the solid dispersion described above, wherein the polymeric carrier is selected from a cellulose polymer, a polyacrylic acid resin polymer, and a mixture thereof.

In one embodiment, the present disclosure provides the solid dispersion described above, wherein the polymeric carrier is selected from cellulose esters, such as cellulose acetate and cellulose acetate phthalate; cellulose ethers, such as ethylcellulose, hydroxypropyl cellulose, and hydroxypropyl methylcellulose (HPMC); and esters of cellulose ethers, such as hydroxypropyl methylcellulose phthalate (HPMCP) and hydroxypropyl methylcellulose acetate succinate (HPMCAS).

In one embodiment, the present disclosure provides the solid dispersion described above, wherein the polymeric carrier is an ester of a cellulose ether, e.g., hydroxypropyl methylcellulose phthalate, e.g., HP-55, HP-55S, or HP-50; and, e.g., hydroxypropyl methylcellulose acetate succinate.

In one embodiment, the present disclosure provides the solid dispersion described above, wherein the polymeric carrier, an ester of a cellulose ether, is preferably hydroxypropyl methylcellulose acetate succinate (HPMCAS), which is a mixture of acetate and succinate of hydroxypropyl methylcellulose and is amphiphilic, wherein an acetyl group provides hydrophobicity and a succinyl group provides hydrophilicity. Due to differences in the contents of acetyl groups and succinyl groups, as well as variations in particle size, HPMCAS usable in the solid dispersion of the present disclosure includes HPMCAS L, HPMCAS LG, HPMCAS M, HPMCAS MG, HPMCAS H, and the like (L, M, and H represent a gradual increase in the acetyl group content and a gradual decrease in the succinyl group content, respectively; G denotes a coarse particle size; F denotes a fine particle size), with HPMCAS LG being more preferred.

In one embodiment, the present disclosure provides the solid dispersion described above, wherein the polymeric carrier is a polyacrylic acid resin polymer selected from a methacrylic acid copolymer and a methacrylate copolymer.

In one embodiment, the present disclosure provides the solid dispersion described above, wherein the polymeric carrier is selected from a butyl methacrylate-dimethylaminoethyl methacrylate-methyl methacrylate copolymer, a methacrylic acid-ethyl acrylate copolymer, a methacrylic acid methyl methacrylate copolymer, an ethyl acrylate-methyl methacrylate-trimethylammonioethyl methacrylate chloride copolymer, an ethyl acrylate-methyl methacrylate copolymer, a methacrylic acid-methyl acrylate-methyl methacrylate copolymer, and a methacrylic acid-butyl acrylate copolymer.

In one embodiment, the present disclosure provides the solid dispersion described above, wherein the polymeric carrier is selected from a butyl methacrylate-dimethylaminoethyl methacrylate-methyl methacrylate (1:2:1) copolymer, a methacrylic acid-ethyl acrylate (1:1) copolymer, a methacrylic acid-methyl methacrylate (1:1 or 1:2) copolymer, an ethyl acrylate methyl methacrylate trimethylammonioethyl methacrylate chloride (1:2:0.1 or 1:2:0.2) copolymer, an ethyl acrylate-methyl methacrylate (2:1) copolymer, a methacrylic acid-butyl acrylate (35:65) copolymer, and a mixture thereof in any ratio.

In one embodiment, the present disclosure provides the solid dispersion described above, wherein the polymeric carrier is selected from Eudragit. In one embodiment, the present disclosure provides the solid dispersion described above, wherein the polymeric carrier is selected from Eudragit E, Eudragit L, Eudragit S, Eudragit RL, and Eudragit RS.

In one embodiment, the present disclosure provides the solid dispersion described above, wherein the polymeric carrier is selected from Eudragit E100, Eudragit EPO, Eudragit L100-55, Eudragit L30D-55, Eudragit L100, Eudragit S100, or a mixture thereof in any ratio.

In one embodiment, the present disclosure provides the solid dispersion described above, wherein the polymeric carrier is selected from a methacrylic acid-ethyl acrylate (1:1) copolymer, and/or a methacrylic acid-methyl methacrylate (1:1) copolymer. In one embodiment, the polymeric carrier in the solid dispersion of the present disclosure is Eudragit E100. In one embodiment, the polymeric carrier in the solid dispersion of the present disclosure is Eudragit L100. In one embodiment, the polymeric carrier in the solid dispersion of the present disclosure is Eudragit L100-55.

In one embodiment, the present disclosure provides the solid dispersion described above, wherein the polymeric carrier is a mixture of a cellulose polymer and a polyacrylic acid resin polymer.

In one embodiment, the present disclosure provides the solid dispersion described above, wherein the polymeric carrier is a mixture of a cellulose polymer and a polyacrylic acid resin polymer, wherein the cellulose polymer is selected from the general or specific cellulose polymers mentioned in the embodiments described above, preferably esters of cellulose ethers, more preferably hydroxypropyl methylcellulose acetate succinate (HPMCAS), and most preferably HPMCAS LG; the polyacrylic acid resin polymer is selected from the general or specific polyacrylic acid resin polymers mentioned in the embodiments described above, e.g., Eudragit, such as Eudragit L100, Eudragit E100, Eudragit L100-55, or a mixture thereof in any ratio, preferably methacrylic acid-ethyl acrylate (1:1) copolymers, e.g., Eudragit L100-55.

In one embodiment, the present disclosure provides the solid dispersion described above, wherein the polymeric carrier is a mixture of the general or specific cellulose polymer described above and the general or specific polyacrylic acid resin polymer described above, wherein the cellulose polymer and the polyacrylic acid resin polymer are in a weight ratio of about 10:1 to 5:1, for example, about 10:1, about 9:1, about 8:1, about 7:1, about 6:1, or about 5:1, preferably about 8:1 to 5:1, about 7:1 to 5:1, 7:1 to 6:1, about 6:1 to 5:1, or any integer or non-integer weight ratio therebetween.

In one embodiment, the present disclosure provides the solid dispersion described above, wherein the EED inhibitor (e.g., compound A) and the polymeric carrier are in a weight ratio (drug loading ratio) of at least about 1:1, such that a uniform dispersion of the active ingredient and the carrier can be obtained by the preparation method of the present disclosure, thereby achieving complete crystal form conversion of the active ingredient into an amorphous state. Such conversion improves the solubility and absorption of the drug, which in turn enhances the bioavailability of the drug after oral administration. Additionally, the solid dispersion prepared in this way is stable per se, showing no aging phenomenon under accelerated conditions and no significant changes in various indicators. Although the higher the content of the carrier, the easier the dissolution and absorption of the active ingredient, a low drug loading rate will increase the formulation mass and raise formulation difficulty. After comprehensive consideration, the drug loading ratio in the solid dispersion of the present disclosure is about 1:1 to 1:5, for example, about 1:1, about 1:1.5, about 1:2, about 1:2.5, about 1:3, about 1:3.5, about 1:4, about 1:4.5, about 1:5, or any integer or non-integer weight ratio therebetween, preferably about 1:1 to 1:2, about 1:1 to 1:3, about 1:1 to 1:4, about 1:2 to 1:3, about 1:2 to 1:4, or about 1:3 to 1:4, and more preferably about 1:3 to 1:4.

In one embodiment, the present disclosure provides the solid dispersion described above, wherein the EED inhibitor (e.g., compound A) accounts for about 10-50 wt% of the solid dispersion or any integer or non-integer point value therebetween, for example, about 5 wt%, about 10 wt%, about 15 wt%, about 20 wt%, about 25 wt%, about 30 wt%, about 35 wt%, about 40 wt%, about 45 wt%, about 50 wt%, or any integer or non-integer weight ratio therebetween.

In one embodiment, the present disclosure provides the solid dispersion described above, wherein the EED inhibitor (e.g., compound A) is present in the solid dispersion in a content range of, for example, about 10-50 wt%, 10-40 wt%, 10-30 wt%, 10-20 wt%, 15-50 wt%, 15-40 wt%, 15-30 wt%, 15-35 wt%, 15-20 wt%, 20-50 wt%, 20-40 wt%, 20-35 wt%, 20-30 wt%, 25-50 wt%, 25-40 wt%, 25-35 wt%, 25-30 wt%, 30-50 wt%, 30-45 wt%, 30-40 wt%, 30-35 wt%, or any integer or non-integer range value therebetween, preferably about 10-40 wt%, 15-40 wt%, 15-35 wt%, 20-50 wt%, 20-40 wt%, or 20-35 wt%.

In one embodiment, the present disclosure provides the solid dispersion described above, wherein the polymeric carrier accounts for about 40 wt% to 90 wt% of the solid dispersion, or any integer or non-integer point value therebetween, e.g., about 40 wt%, about 45 wt%, about 50 wt%, about 55 wt%, about 60 wt%, about 65 wt%, about 70 wt%, about 75 wt%, about 80 wt%, about 85 wt%, or about 90 wt%.

In one embodiment, the present disclosure provides the solid dispersion described above, wherein the polymeric carrier is present in the solid dispersion in a content range of, for example, about 40-90 wt%, 40-80 wt%, 40-70 wt%, 40-60 wt%, 40-50 wt%, 45-90 wt%, 45-80 wt%, 45-70 wt%, 45-60 wt%, 50-90 wt%, 50-80 wt%, 50-70 wt%, 50-60 wt%, 55-90 wt%, 55-80 wt%, 55-70 wt%, 55-65 wt%, 60-90 wt%, 60-80 wt%, 60-70 wt%, 65-90 wt%, 65-80 wt%, 65-75 wt%, 70-90 wt%, 70-80 wt%, or any integer or non-integer point value or range value therebetween, preferably about 40-80 wt%, 50-80 wt%, or 60-80 wt%.

In one embodiment, the present disclosure provides the solid dispersion described above, wherein the solid dispersion consists of an EED inhibitor (e.g., compound A) and a polymeric carrier, wherein the EED inhibitor is the EED inhibitor described generally or preferably above, most preferably compound A; the polymeric carrier is the polymeric carrier described generally or preferably above, more preferably HPMCAS and/or Eudragit (e.g., a methacrylic acid-ethyl acrylate (1:1) copolymer, such as Eudragit L100-55), and most preferably HPMCAS LG. In one embodiment, the weight ratio between the EED inhibitor and the polymeric carrier that form the solid dispersion, as well as the weight ratio of each component to the solid dispersion, are as defined generally or specifically hereinabove, respectively.

In one embodiment, the present disclosure provides the solid dispersion described above, wherein, based on a total weight of the solid dispersion, the EED inhibitor (e.g., compound A) has a content range of 20-50 wt% and the polymeric carrier has a content range of 50-80 wt%; preferably, the EED inhibitor (e.g., compound A) has a content range of 20-40 wt% and the polymeric carrier has a content range of 60-80 wt%.

According to the aforementioned solid dispersion provided in the present disclosure, the active ingredient EED inhibitor (e.g., compound A) is confirmed to be in an amorphous state and achieve complete crystal form conversion by methods such as PLM and/or XRPD and/or DSC.

The aforementioned solid dispersion provided in the present disclosure is stable, i.e., has chemical stability and solid stability, in conditions selected from the following: high temperature (60 °C), high humidity (25 °C/RH90%), acceleration conditions (40 °C/RH75%), refrigeration (2-8 °C), grinding (5-15 min/room temperature), grinding and refrigeration (5-15 min/2-8 °C), and tableting (room temperature or refrigeration). The active ingredient EED inhibitor is confirmed to be in an amorphous state without aging and no significant changes in the content of the active ingredient and impurities by methods such as PLM and/or XRPD and/or DSC.

The aforementioned solid dispersion provided in the present disclosure can maintain stable solubility in fasted state simulated intestinal fluid (FaSSIF) at pH 6.5 for at least 24 h, and its solubility is at least about 10 times, for example, about 10 times, about 20 times, about 25 times, about 30 times, about 35 times, about 40 times, about 50 times, or about 60 times, that of the parent drug of the EED inhibitor (e.g., compound A) under the same conditions.

In one embodiment, the present disclosure provides the solid dispersion described above comprising an EED inhibitor (e.g., compound A) and polymeric carriers, cellulose ether-esters (e.g., HPMCAS) and polyacrylic acid resins (e.g., Eudragit, such as a methacrylic acid-ethyl acrylate (1:1) copolymer, e.g., Eudragit L100-55), wherein the EED inhibitor, a drug loading thereof, the cellulose ether-ester and polyacrylic acid resin polymeric carriers, a content thereof, and a weight ratio therebetween are as generally and specifically defined above for the corresponding characteristics, the solid dispersion maintains stable solubility in fed state simulated intestinal fluid (FeSSIF) at pH 5.8 for at least 24 h, and the EED inhibitor remains free from crystal form conversion for at least 24 h.

In one embodiment, an EED inhibitor (e.g., compound A) and a cellulose ether-ester polymeric carrier (e.g., HPMCAS) can be first prepared into a solid dispersion according to the method of the present disclosure, and then a polyacrylic acid resin (e.g., Eudragit, such as methacrylic acid-ethyl acrylate (1:1) copolymer, e.g., Eudragit L100-55) polymer is added. The mixture comprising the solid dispersion prepared in this way also enables the active ingredient EED inhibitor to be in an amorphous state, has the chemical stability and solid stability described above, can maintain stable solubility in fasted state simulated intestinal fluid (FaSSIF) at pH 6.5 for at least 24 h, which is much higher than the solubility of the parent drug of the EED inhibitor under the same conditions, maintains stable solubility in fed state simulated intestinal fluid (FeSSIF) at pH 5.8 for at least 24 h, and remains free from crystal form conversion for at least 24 h.

The aforementioned solid dispersion provided in the present disclosure has been shown in animal pharmacokinetic experiments (e.g., the pharmacokinetic study in beagle dogs as illustrated in the Example) that, when administered at the same dose, the *in vivo* exposure of the EED inhibitor solid dispersion of the present disclosure is comparable to that of the EED inhibitor solution, and the AUC value reaches at least 80% of that of the solution.

The aforementioned solid dispersion provided in the present disclosure can be directly used, or prepared into different dosage forms according to therapeutic or prophylactic needs.

**In a second aspect,** the present disclosure provides a preparation method for the EED inhibitor solid dispersion of the present disclosure. The solid dispersion of the present disclosure can be obtained by widely known preparation methods such as a melt method, a solvent method, a solvent-melt method, a spray drying method, a fluidized bed drying method, and a melt-extrusion method.

In a specific embodiment, the present disclosure preferably prepares an EED inhibitor solid dispersion by a spray drying method, which comprises the following steps:
(1) dissolving an EED inhibitor (e.g., compound A) in an organic solvent, then adding a polymeric carrier, and dissolving by stirring to prepare a spray drying solution;
(2) spray drying the solution obtained in step (1) to remove the organic solvent.

In one embodiment, the method for preparing an EED inhibitor solid dispersion described above further comprises step (3): reduced-pressure drying the spray dried powder obtained in step (2).

In step (1), the organic solvent used is not strictly limited, but should be one that can fully dissolve the EED inhibitor, for example, but not limited to, methanol, absolute ethanol, isopropanol, isobutanol, ethyl acetate, acetone, dichloromethane, n-hexane, n-heptane, tetrahydrofuran, acetonitrile, benzene, toluene, xylene, dimethyl sulfoxide, dimethylformamide, or N,N-dimethylacetamide, preferably dichloromethane, methanol, or a mixture thereof in any ratio, and more preferably, a mixed solvent of dichloromethane/methanol of about 2:1 to 3:1.

In step (1), an amount of the organic solvent used should be an amount sufficient to fully dissolve the EED inhibitor; a solid content concentration of a suitable solution is not greater than 120 mg/mL, preferably in a range of 20-80 mg/mL, and more preferably in a range of 40-60 mg/mL.

In step (1), the form of the EED inhibitor is not strictly limited, and an amorphous form or a crystal form may be used. The EED inhibitor in different crystal forms can be converted into an amorphous form in the solid dispersion system of the present disclosure.

In step (2), the conditions and means for drying are not strictly limited as long as the organic solvent can be removed. In one embodiment, the drying in step (2) is performed by the spray drying method, and those skilled in the art can determine various parameters of the spray drying, for example, an inlet air temperature of the spray drying can be controlled in a range of 65-95 °C, e.g., at 70 °C. In a specific embodiment, the spray drying is performed in the absence of light.

In one embodiment, the reduced-pressure drying in step (3) is optionally performed after step (2) to further remove the organic solvent; conditions for the reduced-pressure drying are not strictly limited and can be determined by those skilled in the art. In a specific embodiment, the reduced-pressure drying is performed at a temperature of 25-50 °C (e.g., 50 °C) and a reduced pressure of 0 Mpa to -0.1 Mpa (e.g., -0.1 Mpa) for a suitable period of time, for example, but not limited to, 2-5 h, such as 4 h.

In the preparation method for the solid dispersion according to this aspect of the present disclosure, a yield of the solid dispersion reaches at least 75%, and compactibility is good.

**In a third aspect,** the present disclosure provides a solid dispersion composition comprising the solid dispersion of the present disclosure and an excipient, wherein the solid dispersion is as generally or specifically defined herein for the solid dispersion of the present disclosure, and the excipient is selected from one or more of a diluent, a flavoring agent, a surfactant, a filler, a binder, a disintegrant, a lubricant, a glidant/antiadherent, a stabilizer, an emulsifier, a coating agent, and/or a release modifier.

In one embodiment, the present disclosure provides a solid dispersion composition, which may be in the form of a tablet, a capsule, a granule, a powder, a pill, or the like.

**In a fourth aspect,** the present disclosure provides a preparation method for the solid dispersion composition of the present disclosure described above, which comprises the following steps:
(1) uniformly mixing the EED inhibitor solid dispersion of the present disclosure, a filler, a binder, a disintegrant, a stabilizer, and/or a glidant;
(2) optionally granulating the mixture of step (1);
(3) optionally fully mixing the granule prepared in step (2) with a lubricant.

In one embodiment, the composition prepared above may be prepared in the form of a pill, a granule, a powder, or the like, or may be further compressed in the form of a tablet, or the resulting mixture or granule is directly filled into a capsule shell to prepare a capsule.

**In a fifth aspect,** the present disclosure provides an oral formulation of an EED inhibitor (e.g., compound A), which comprises the solid dispersion of the present disclosure or the solid dispersion composition of the present disclosure, and optionally an excipient.

In one embodiment, the present disclosure provides an oral formulation of an EED inhibitor (e.g., compound A), which is an oral solid formulation selected from a tablet, a capsule, a granule, a powder, a lozenge, and a pill, preferably a tablet and a capsule, and more preferably a tablet.

In one embodiment, the present disclosure provides an oral formulation of an EED inhibitor (e.g., compound A), which comprises the solid dispersion of the present disclosure, and further comprises one or more of a filler, a stabilizer, a binder, a disintegrant, a lubricant, a glidant, and a coating agent.

In one embodiment, the present disclosure provides an oral formulation of an EED inhibitor (e.g., compound A), which comprises the solid dispersion of the present disclosure, and further comprises a filler, a binder, a disintegrant, and a lubricant.

In one embodiment, the present disclosure provides an oral formulation of an EED inhibitor (e.g., compound A), which comprises the solid dispersion of the present disclosure, and further comprises a filler, a binder, a disintegrant, a lubricant, and a glidant.

In one embodiment, the present disclosure provides an oral formulation of an EED inhibitor (e.g., compound A), which comprises the solid dispersion of the present disclosure, and further comprises a filler, a binder, a disintegrant, a lubricant, a stabilizer, and a glidant.

In one embodiment, the present disclosure provides an oral formulation of an EED inhibitor (e.g., compound A), which comprises the solid dispersion of the present disclosure, and further comprises a filler, a binder, a disintegrant, a lubricant, a stabilizer, a glidant, and a coating agent.

In one embodiment, the present disclosure provides an oral formulation of an EED inhibitor (e.g., compound A), wherein the EED inhibitor has a content of about 5-40 wt% or any integer or non-integer point value or range value therebetween, for example, but not limited to, about 5-30 wt%, about 5-20 wt%, about 10-40 wt%, about 10-30 wt%, about 10-20 wt%, about 15-40 wt%, about 15-30 wt%, about 15-25 wt%, about 20-40 wt%, about 20-30 wt%, about 25-40 wt%, about 25-35 wt%, or about 30-40 wt%, preferably about 10-30%, and more preferably about 10-20%, by weight of the oral formulation.

In one embodiment, the present disclosure provides an oral formulation of an EED inhibitor (e.g., compound A), wherein the EED inhibitor has an amount in a unit formulation of about 5-500 mg, for example, but not limited to, about 5-400 mg, about 5-300 mg, about 5-200 mg, about 10-500 mg, about 10-400 mg, or about 10-200 mg, preferably about 10-200 mg, e.g., about 200 mg, about 150 mg, about 100 mg, about 75 mg, about 50 mg, about 25 mg, about 15 mg, or about 10 mg, and more preferably about 10 mg, about 50 mg, or about 200 mg.

In one embodiment, the present disclosure provides an oral formulation of an EED inhibitor (e.g., compound A), wherein the solid dispersion accounts for about 10-80% by weight of the oral formulation, or any integer or non-integer point value or range value therebetween, for example, but not limited to, about 10-70%, about 10-50%, about 20-80%, about 20-70%, about 20-60%, about 30-80%, about 30-70%, about 30-50%, about 40-80%, about 40-70%, about 50-80%, or about 50-70%, preferably about 30-80%, about 40-80%, about 40-70%, about 50-80%, or about 50-70%, and more preferably about 50-70%.

In one embodiment, the present disclosure provides an oral formulation of an EED inhibitor (e.g., compound A), wherein the filler may be selected from lactose, sucrose, calcium phosphate, calcium hydrogen phosphate, anhydrous calcium hydrogen phosphate, microcrystalline cellulose, silicified microcrystalline cellulose, pregelatinized starch, starch, mannitol, and the like; preferably, the filler is microcrystalline cellulose; the filler is used in an amount of about 10-80% by weight of the oral formulation, or any integer or non-integer point value or range value therebetween, for example, but not limited to, about 10-70%, about 10-60%, about 10-50%, about 10-40%, about 10-30%, about 15-35%, about 15-40%, about 20-80%, about 20-70%, about 20-60%, about 20-50%, about 20-40%, about 30-80%, about 30-70%, about 30-60%, about 30-50%, or about 30-40%, preferably about 10-60%, about 15-40%, about 20-60%, about 20-40%, or about 20-30%, more preferably about 20-40%, and most preferably about 20-30%.

In one embodiment, the present disclosure provides an oral formulation of an EED inhibitor (e.g., compound A), wherein the binder may be selected from polyvinylpyrrolidone, starch, methylcellulose, hydroxypropyl cellulose, hydroxypropyl methylcellulose, alginate, and the like; preferably, the binder is selected from polyvinylpyrrolidone and hydroxypropyl methylcellulose; the binder is used in an amount of 0.5-10% by weight of the oral formulation, or any integer or non-integer point value or range value therebetween, for example, but not limited to about, 0.5-9%, about 0.5-8%, about 0.5-5%, about 1-10%, about 1-9%, about 1-8%, about 1-5%, about 1-3%, about 1.5-2.5%, about 2-10%, about 2-8%, about 2-5%, or about 2-4%, preferably about 0.5-5%, about 1-3%, or about 1.5-2.5%, more preferably about 1-3%, and most preferably about 1.5-2.5%.

In one embodiment, the present disclosure provides an oral formulation of an EED inhibitor (e.g., compound A), wherein the disintegrant may be selected from croscarmellose sodium, crospovidone, sodium carboxymethyl starch, starch, pregelatinized starch, and the like; preferably, the disintegrant is croscarmellose sodium; the disintegrant is used in an amount of 1-20% by weight of the oral formulation, or any integer or non-integer point value or range value therebetween, for example, but not limited to, about 1.0%, about 2.0%, about 5.0%, about 7.0%, about 10%, about 15%, about 20%, about 1-10%, about 1-8%, about 2-10%, about 2-8%, about 2-7%, about 3-10%, about 3-8%, about 3-7%, about 4-10%, or about 4-8%, preferably about 1-10%, about 2-8%, or about 3-7%, more preferably about 2-8%, and most preferably about 3-7%.

In one embodiment, the present disclosure provides an oral formulation of an EED inhibitor (e.g., compound A), wherein the lubricant may be selected from stearic acid, magnesium stearate, calcium stearate, zinc stearate, palmitic acid, glyceryl palmitostearate, talc, carnauba wax, sodium lauryl sulfate, sodium stearyl fumarate, and the like; preferably, the lubricant is selected from stearic acid, magnesium stearate, calcium stearate, and zinc stearate; the lubricant is used in an amount of 0.1-5% by weight of the oral formulation, or any integer or non-integer point value or range value therebetween, for example, but not limited to, about 0.1%, about 0.5%, about 1.0%, about 1.5%, about 2.0%, about 2.5%, about 3.0%, about 3.5%, about 4.0%, about 4.5%, about 5.0%, about 0.5-5%, about 0.5-4%, about 0.5-3%, about 0.5-2%, about 0.5-2.5%, about 0.5-1.5%, about 1-5%, about 1-4%, about 1-3%, about 1-2.5%, or about 1-2%, preferably about 0.5-3%, about 0.5-2%, about 0.5-1.5%, about 1-3%, or about 1-2%, more preferably about 0.5-2%, and most preferably about 0.5-1.5%.

In one embodiment, the present disclosure provides an oral formulation of an EED inhibitor (e.g., compound A), which further comprises a stabilizer, wherein the stabilizer may be selected from hydroxypropyl methylcellulose, sodium dodecyl sulfate, a polyacrylic acid resin polymer, and the like, preferably polyacrylic acid resin polymers, for example, the general or specific polyacrylic acid resin polymers mentioned in the embodiments described above, such as Eudragit, e.g., Eudragit L100, Eudragit E100, Eudragit L100-55, or a mixture thereof in any ratio, and preferably a methacrylic acid-ethyl acrylate (1:1) copolymer, e.g., Eudragit L100-55; the stabilizer is used in an amount of 1-20% by weight of the oral formulation, or any integer or non-integer point value or range value therebetween, for example, but not limited to, about 1.0%, about 2.0%, about 3.0%, about 5.0%, about 7.0%, about 10%, about 12%, about 15%, about 1-10%, about 1-8%, about 2-10%, about 2-8%, about 3-10%, about 3-8%, about 4-10%, about 4-8%, about 5-10%, or about 5-8%, preferably about 1-10%, about 5-10%, about 2-8%, or about 4-8%, more preferably about 2-8%, and most preferably about 4-8%.

In one embodiment, the present disclosure provides an oral formulation of an EED inhibitor (e.g., compound A), which further comprises a glidant, wherein the glidant may be selected from talc, silicon dioxide, microsilica gel (colloidal silica), and silicon dioxide in other pharmaceutically acceptable forms; preferably, the glidant is colloidal silica; the glidant is used in an amount of 0-5% by weight of the oral formulation, or any integer or non-integer point value or range value therebetween, for example, but not limited to, 0%, about 0.5%, about 1.0%, about 1.5%, about 2.0%, about 2.5%, about 3.0%, about 3.5%, about 4.0%, about 4.5%, about 0.5-5%, 0.5-4%, 0.5-3%, 0.5-2%, 0.5-1.5%, 1-3%, or 1-2%, preferably about 0.5-4%, 0.5-3%, 0.5-2%, or 0.5-1.5%, more preferably about 0.5-2%, and most preferably about 0.5-1.5%.

In one embodiment, the present disclosure provides the oral formulation of an EED inhibitor (e.g., compound A) described above, such as a tablet, and the oral formulation comprises:
1) about 10-80 wt% solid dispersion as generally or specifically defined herein comprising about 5-500 mg of an EED inhibitor (e.g., compound A) as generally or specifically defined herein and a polymeric carrier as generally or specifically defined herein;
2) about 10-80 wt% filler as generally or specifically defined herein;
3) about 0.5-10 wt% binder as generally or specifically defined herein;
4) about 1-20 wt% disintegrant as generally or specifically defined herein; and
5) about 0.1-5 wt% lubricant as generally or specifically defined herein.

Preferably, the oral formulation comprises:
1) about 50-80 wt% solid dispersion as generally or specifically defined herein comprising about 10-500 mg of an EED inhibitor (e.g., compound A) as generally or specifically defined herein and a polymeric carrier as generally or specifically defined herein;
2) about 20-60 wt% filler as generally or specifically defined herein;
3) about 0.5-5 wt% binder as generally or specifically defined herein;
4) about 1-10 wt% disintegrant as generally or specifically defined herein; and
5) about 0.5-3 wt% lubricant as generally or specifically defined herein.

More preferably, the oral formulation comprises:
1) about 30-80 wt% solid dispersion as generally or specifically defined herein comprising about 10-200 mg of an EED inhibitor (e.g., compound A) as generally or specifically defined herein and a polymeric carrier as generally or specifically defined herein;
2) about 20-40 wt% filler as generally or specifically defined herein;
3) about 1-3 wt% binder as generally or specifically defined herein;
4) about 2-8 wt% disintegrant as generally or specifically defined herein; and
5) about 0.5-2 wt% lubricant as generally or specifically defined herein.

Most preferably, the oral formulation comprises:
1) about 50-70 wt% solid dispersion as generally or specifically defined herein comprising about 10-200 mg of an EED inhibitor (e.g., compound A) as generally or specifically defined herein and a polymeric carrier as generally or specifically defined herein;
2) about 20-30 wt% filler as generally or specifically defined herein;
3) about 1.5-2.5 wt% binder as generally or specifically defined herein;
4) about 3-7 wt% disintegrant as generally or specifically defined herein; and
5) about 0.5-1.5 wt% lubricant as generally or specifically defined herein.

The general, preferred, more preferred, or most preferred oral formulation described above further optionally comprises:
6) about 1-20 wt%, preferably about 1-10 wt%, more preferably about 2-8 wt%, and most preferably about 4-8 wt% stabilizer as generally or specifically defined herein; and/or
the general, preferred, more preferred, or most preferred oral formulation described above further optionally comprises:
7) about 0-5 wt%, preferably about 0.5-3 wt%, more preferably about 0.5-2 wt%, and most preferably about 0.5-1.5 wt% glidant as generally or specifically defined herein.

It should be noted that in the oral formulations of an EED inhibitor (e.g., compound A) of the present disclosure as generally or preferably defined above, the solid dispersion, the selection of each component therefor, the content of each component, and the ratio of the components to each other (e.g., the drug loading ratio and the ratio between polymeric carriers) are all as defined in the first aspect of the present disclosure above, i.e., the formulations encompass both solid dispersion technical solutions constituted by the general or preferred definitions of each technical feature, as well as solid dispersion technical solutions constituted by any combination of the general or preferred definitions of the various technical features.

In one embodiment, the present disclosure provides the oral formulation of an EED inhibitor described above, such as a tablet, wherein the EED inhibitor is compound A or a pharmaceutically acceptable salt or solvate thereof, and the polymeric carrier in the solid dispersion is hydroxypropyl methylcellulose acetate succinate (HPMCAS).

In one embodiment, the present disclosure provides the oral formulation of an EED inhibitor described above, such as a tablet, wherein the EED inhibitor is compound A or a pharmaceutically acceptable salt or solvate thereof, and the polymeric carrier in the solid dispersion is a mixture of cellulose ether-ester (e.g., HPMCAS) and polyacrylic acid resin (e.g., Eudragit, e.g., methacrylic acid-ethyl acrylate (1:1) copolymer, e.g., Eudragit L100-55) polymers as generally or specifically defined herein.

In one embodiment, the present disclosure provides the oral formulation of an EED inhibitor described above, such as a tablet, wherein the EED inhibitor is compound A or a pharmaceutically acceptable salt or solvate thereof, and the polymeric carrier in the solid dispersion is a polyacrylic acid resin (e.g., Eudragit, e.g., methacrylic acid-ethyl acrylate (1:1) copolymer, e.g., Eudragit L100-55) polymer as generally or specifically defined herein.

In one embodiment, the present disclosure provides the oral formulation of an EED inhibitor (e.g., compound A) described above, such as a tablet, wherein the filler is microcrystalline cellulose, the binder is hydroxypropyl methylcellulose, the disintegrant is croscarmellose sodium, the lubricant is magnesium stearate, the stabilizer is a methacrylic acid-ethyl acrylate (1:1) copolymer (e.g., Eudragit L100-55), and the glidant is colloidal silica.

In one embodiment, the present disclosure provides the oral formulation of an EED inhibitor (e.g., compound A) described above, such as a tablet, wherein the tablet may be a coated tablet, wherein the coating agent is well known to those skilled in the art, for example, but not limited to, sodium carboxymethyl cellulose, cellulose acetate, cellulose acetate phthalate, methylcellulose, ethylcellulose, hydroxypropyl cellulose, hydroxypropyl methylcellulose, hydroxypropyl methylcellulose phthalate, a methacrylic acid copolymer, gelatin, a pharmaceutical glaze, polyethylene glycol, polyvinyl acetate phthalate, sodium dodecyl sulfate, shellac, sucrose, titanium dioxide, carnauba wax, zein, Opadry, and the like, or a mixture thereof, or a conventional film coating premix well known to those skilled in the art; the coating agent is used in an amount of 0-10% by weight of the oral formulation, or any range therebetween, for example, but not limited to, about 0.5%, about 0.6%, about 0.7%, about 0.8%, about 0.9%, about 1.0%, about 1.5%, about 2.0%, about 2.5%, about 3.0%, about 3.5%, about 4.0%, about 4.5%, about 5.0%, about 6.0%, about 7.0%, about 8.0%, about 9.0%, or about 10%, preferably about 1-5% or 2-4%, and more preferably about 2-4%.

The aforementioned oral formulation of an EED inhibitor (e.g., compound A) provided in the present disclosure, such as a tablet, dissolves rapidly and completely. The dissolution of the active ingredient in an aqueous medium containing 0.15% sodium dodecyl sulfate (SDS) at pH 6-7 is maintained at about 75% or higher, preferably about 80% or higher, and more preferably 85% or higher at 30 min, and is maintained at about 85% or higher, preferably about 90% or higher at 45 min.

The aforementioned oral formulation of an EED inhibitor (e.g., compound A) provided in the present disclosure is a tablet with tablet hardness of about 50-400 N. In a specific embodiment, for a 10 mg tablet, the hardness is about 30-90 N, preferably about 30-80 N. In a specific embodiment, for a 50 mg tablet, the hardness is about 50-200 N, preferably about 70-150 N. In a specific embodiment, for a 200 mg tablet, the hardness is about 150-400 N, preferably about 200-360 N.

The aforementioned oral formulation of an EED inhibitor (e.g., compound A) provided in the present disclosure is a tablet with tablet friability less than about 1 wt%, preferably less than about 0.5 wt%, such as less than about 0.2 wt%, and more preferably less than about 0.1 wt%.

The aforementioned oral formulation of an EED inhibitor (e.g., compound A) provided in the present disclosure is a tablet with a disintegration time limit of less than about 15 min, preferably less than about 5 min, such as about 0.5-5 min.

For the aforementioned oral formulation of an EED inhibitor (e.g., compound A) provided in the present disclosure, the mixture of the raw and auxiliary materials has good stability and shows no significant change in appearance and no significant increase in related substances after being left to stand under conditions of high temperature (60 °C), high humidity (25 °C/RH90%±5%), and acceleration (40 °C±2 °C/RH75%±5%) and in the dark for 0/5/10/30 days.

The aforementioned oral formulation of an EED inhibitor (e.g., compound A) provided in the present disclosure is a tablet, and the finished formulation packaged in a conventional packaging material has good stability. After being left to stand under conditions of 40 °C±2 °C/RH75%±5%, 30 °C±2 °C/RH65%±5%, and 25 °C±2 °C/RH60%±5% for 1 month, the test sample shows no significant change in appearance, water content, related substances, content, and dissolution.

**In a sixth aspect,** the present disclosure provides a preparation method for the oral formulation in the tablet form of the present disclosure described above, which comprises the following steps:
(1) uniformly mixing the EED inhibitor solid dispersion of the present disclosure, a filler, a binder, a disintegrant, a stabilizer, and/or a glidant in a mixer;
(2) granulating the mixture of step (1);
(3) fully mixing the granule prepared in step (2) with a lubricant;
(4) tableting the mixture prepared in step (3).

In a preferred embodiment, in step (1), the EED inhibitor solid dispersion of the present disclosure, a filler, a stabilizer, a binder, a disintegrant, and a glidant are uniformly mixed.

In a preferred embodiment, the mixture of step (1) is sieved and then mixed.

In a preferred embodiment, the sieved mixture of step (1) is mixed again.

In a preferred embodiment, the mixture of step (1) is thoroughly mixed with a portion of a lubricant before the granulation of step (2) is performed, wherein the portion of the lubricant added may suitably be about 40-70%, preferably about 50-70%, of the total weight of the lubricant in the formula. The addition of the portion of the lubricant at this stage helps to further mitigate adhesion during subsequent granulation and sticking during tableting.

In step (2), the conditions and means for granulation are not strictly limited, and the granulation may be wet granulation or dry granulation or melt granulation.

In a preferred embodiment, the granulation in step (2) is performed by dry granulation, which may be performed by conventional methods known in the art, for example, by applying a roller compaction method, see, e.g., Pharmaceutics, edited by Fang Liang, 3rd Edition, July 2015, China Medical Science and Technology Press.

In a preferred embodiment, the pressure of the dry granulation may be 6-8 Kpa, preferably 4-6 KPa. Adopting the pressure range can further reduce the adhesion of the dry granulated material to the compression rollers, further improve the flowability of the prepared granules, and further reduce the sticking during tableting.

The tablet prepared according to the method described above has friability of less than 1%, preferably less than about 0.5%, a disintegration time limit of less than 15 min, preferably less than 10 min or less than 5 min, and dissolution reaching no less than 75% at 30 min and no less than 85% at 45 min in an SDS solution at pH 6.8.

In one embodiment, the preparation method for the oral formulation in the tablet form according to this aspect of the present disclosure further comprises step (5): coating the tablet prepared in step (4) with a coating material.

In step (5), the coating material used is not particularly limited, but should have no significant effect on the content and dissolution of the tablet and provide a light-shielding function, thereby further enhancing the storage stability of the tablet formulation.

In one embodiment, the coating material in step (5) accounts for about 1-5%, preferably 2-4%, of the total weight of the formula of the oral tablet, and increases the weight of the tablet core by about 1-5%, preferably 2-4%.

It should be noted that in the preparation methods according to the second aspect, the fourth aspect, and the sixth aspect of the present disclosure described above, the selections and amounts of the EED inhibitor (e.g., compound A), the polymeric carrier, the solid dispersion, and various excipients are as generally, specifically, or preferably defined above for the corresponding technical solutions. Most preferably, the EED inhibitor is compound A or a pharmaceutically acceptable salt or solvate thereof; the polymeric carrier is hydroxypropyl methylcellulose acetate succinate (HPMCAS) and/or a methacrylic acid-ethyl acrylate (1:1) copolymer (e.g., Eudragit L100-55); the filler is microcrystalline cellulose; the stabilizer is a methacrylic acid-ethyl acrylate (1:1) copolymer (e.g., Eudragit L100-55); the binder is hydroxypropyl methylcellulose; the disintegrant is croscarmellose sodium; the lubricant is magnesium stearate; the glidant is colloidal silica.

**In a seventh aspect,** the present disclosure provides use of the solid dispersion, the solid dispersion composition, or the oral formulation of the present disclosure described above in treating or preventing a disease (e.g., cancer or tumor) where inhibition of EED provides a benefit.

**In an eighth aspect,** the present disclosure provides use of the solid dispersion, the solid dispersion composition, or the oral formulation of the present disclosure described above in preparing a medicament for treating or preventing a disease (e.g., cancer or tumor) where inhibition of EED provides a benefit.

**In a ninth aspect,** the present disclosure provides a method for treating or preventing a disease (e.g., cancer or tumor) where inhibition of EED provides a benefit in a patient, which comprises administering a therapeutically effective amount of the solid dispersion, the solid dispersion composition, or the oral formulation of the present disclosure to a subject in need.

It should be noted that the "disease where inhibition of EED provides a benefit" described in the seventh to ninth aspects of the present disclosure described above is selected from cancer and proliferative diseases, inflammatory diseases, septicemia, autoimmune diseases, and viral infections, preferably cancer and proliferative diseases. For specific examples, see the corresponding contents of CN114127073A.

In one embodiment, the "disease where inhibition of EED provides a benefit" described in the seventh to ninth aspects of the present disclosure described above is cancer selected from acute monocytic leukemia, acute myeloid leukemia, chronic myeloid leukemia, chronic lymphocytic leukemia, mixed lineage leukemia, NUT-midline carcinoma, multiple myeloma, small cell lung cancer, non-small cell lung cancer, neuroblastoma, Burkitt lymphoma, cervical cancer, esophageal cancer, ovarian cancer, colorectal cancer, prostate cancer, breast cancer, bladder cancer, ovarian cancer, glioma, sarcoma, esophageal squamous cell carcinoma, and papillary thyroid carcinoma.

According to the technical solutions of the present disclosure described above, the poorly water-soluble EED inhibitor active ingredient, e.g., compound A, is prepared into a solid dispersion with a specific type of polymeric carrier, and the active ingredient of the drug maintains a stable amorphous state in the solid dispersion environment, and meanwhile, the solubility and dissolution of the drug in gastrointestinal fluid are improved, so that the oral bioavailability of the drug is improved. Tablets prepared using the solid dispersion in combination with specific excipient combinations exhibit good formulation performances and simultaneously achieve excellent levels in friability, hardness, disintegration time limit, and dissolution. The preparation method employed features a simple process, easily controllable parameters, and good reproducibility, making it easy for industrial production.

### BRIEF DESCRIPTION OF THE DRAWINGS

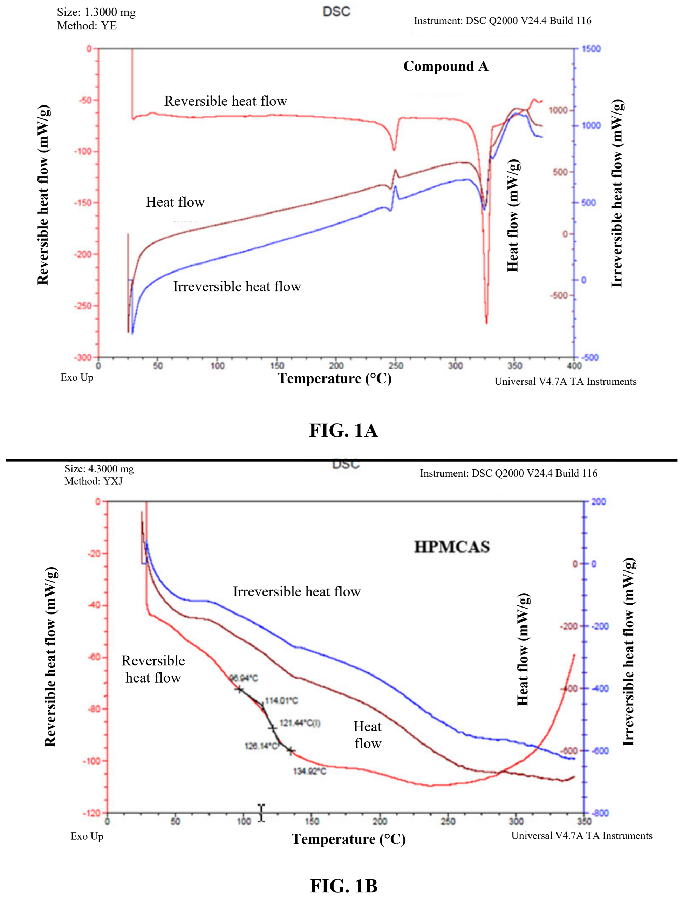

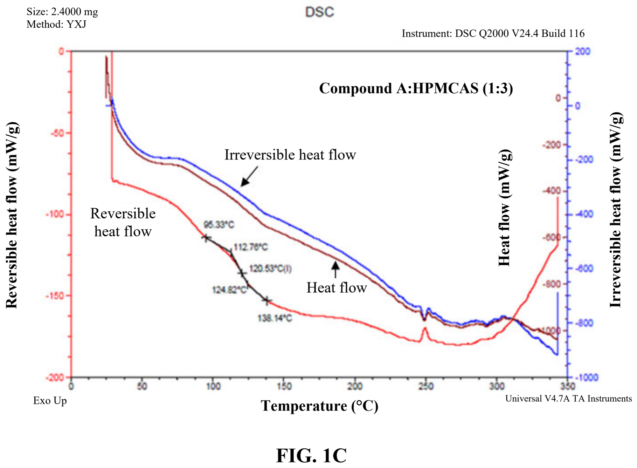

FIG. 1: DSC patterns of compound A (also referred to as API hereinafter) (FIG. 1A), a representative polymeric carrier (FIG. 1B), and a mixture of compound A and a representative polymeric carrier (drug loading ratio: 1:3) (FIG. 1C).
FIG. 2 shows PLM images of solid dispersions prepared with different carriers (hydroxypropyl methylcellulose acetate succinate-LG, copovidone VA64, or hydroxypropyl cellulose EXF) and API.
FIG. 3A shows XRPD patterns of solid dispersions prepared with different carriers (hydroxypropyl methylcellulose acetate succinate-LG, copovidone VA64, or hydroxypropyl cellulose EXF) and API; FIG. 3B shows XRPD patterns of solid dispersions prepared with different carriers (Eudragit L100-55 or Eudragit L100-55 + HPMCAS-LG) and API; FIG. 3C shows XRPD patterns of solid dispersions with different drug loading ratios (API:HPMCAS-LG).
FIGs. 4A-4D show DSC patterns of solid dispersions with different drug loading ratios (API:HPMCAS-LG).
FIG. 5A shows the dynamic solubility of compound A solid dispersions prepared with different polymeric carriers in an FaSSIF-V2 solution; FIG. 5B shows the dynamic solubility of solid dispersions with different drug loading ratios (API:HPMCAS-LG) in an FeSSIF-V2 solution with the additional addition of Eudragit L100-55.
FIG. 6 shows the pharmacokinetic properties of a solid dispersion of the present disclosure orally administered to beagle dogs.
FIG. 7 shows the dissolution properties of an oral solid formulation of the present disclosure.

### DETAILED DESCRIPTION

The present disclosure is further illustrated and explained by the following examples, which, however, are not intended to limit the protection scope of the present disclosure.

Unless otherwise specified, the auxiliary materials and reagents used in the examples are all commercially available, and their quality standards and amounts all conform to the standards of Chinese Pharmacopoeia; the EED inhibitor used in the present disclosure can be prepared by reference to the method described in CN114127073A, for example, compound A or the pharmaceutically acceptable salt thereof can be prepared by reference to the method of Example 73 therein.

### General Experimental Methods

**1.** X-ray powder diffraction (XRPD): A D8 Advance instrument equipped with a Lynxeye detector was used. Patterns were recorded under the following conditions: X-ray (Cu, Kα (Å): 1.54), 45 kV/40 mA, step size (°2θ) of 0.02, scanning rate (s/step) of 0.1, and scanning range (°2θ) of 3-40.
**2.** Polarized light microscopy (PLM): An Axiolab5 instrument was used. An appropriate amount of the sample to be tested was placed on a glass slide at room temperature, a small amount of paraffin was added dropwise to disperse the sample, a cover slip was laid on top to disperse the sample, and the cover slip was then removed; the sample was observed under a 20× objective lens (at a magnification of 200×).
**3.** Modulated differential scanning calorimetry (mDSC): Discovery DSC 2500 was used. A linear heating rate (10 °C/min) was applied, and patterns were recorded from 25 °C under nitrogen atmosphere.
**4.** Active ingredient content determination: A high performance liquid chromatograph (HPLC) equipped with a UV detector or diode array detector and an autosampler, with a chromatographic column of Waters Xbridge C18 (4.6 × 150 mm, 3.5 µm, column temperature of 30 °C), mobile phases (A:B = 40:60, A: a 0.05% aqueous phosphoric acid solution, B: a solution of 0.05% phosphoric acid in methanol:acetonitrile (1:9)), and a flow rate of 0.8 mL/min, was used. The sample was diluted with methanol, and 10 µL of the sample was injected. The change in absorbance at UV 249 nm over time was recorded.
**5.** Determination of dissolution, tablet friability, hardness, and disintegration time limit: The determination was conducted according to conventional methods in the art, for example, by referring to the corresponding determination methods described in the Chinese Pharmacopoeia, 2015 Edition.
**6.** Fasted state simulated intestinal fluid (FaSSIF), fed state simulated intestinal fluid (FeSSIF), and simulated gastric fluid (SGF) can be purchased commercially or formulated as follows:
Formulation of FaSSIF-V2 solution: 1.39 g of sodium hydroxide, 2.22 g of maleic acid, and 4.01 g of sodium chloride were weighed out, and the volume was made up to 1000 mL. Sonication was performed for dissolution. The pH was adjusted to 6.5 for later use. 1.79 g of FaSSIF-V2 powder was added, and the mixture was dissolved for later use.
Formulation of FeSSIF-V2 solution: 1.64 g of sodium hydroxide, 3.19 g of maleic acid, and 3.67 g of sodium chloride were weighed out and dissolved in 500 mL of pure water, and the pH was adjusted to 5.8. 4.88 g of an FeSSIF-V2 solution was added, and sonication was performed until the mixture was completely dissolved for later use.
Formulation of simulated gastric fluid (SGF): 0.96 mL of concentrated hydrochloric acid was taken, and 150 mL of water and 2.5 g of pepsin were added. The mixture was uniformly mixed and then diluted to 250 mL with water for later use.

7. Preparation of solid dispersion: A formula amount of compound A was weighed out and added to a solvent (e.g., a mixed solvent of dichloromethane and methanol), and the mixture was stirred until dissolved. A formula amount of carrier substance was added. The mixture was stirred until dissolved and subjected to spray drying in a spray dryer with selected appropriate parameters, such as inlet air temperature, inlet air amount, liquid feeding speed, and pressure, to give the solid dispersion of the present disclosure. Spray drying equipment suitable for use in the present disclosure includes, but is not limited to, corresponding equipment produced by Niro GEA Process Engineering Inc., Buchi Labortechnik AG, ProCept, and SPX ANHYDROUS. The present disclosure illustratively uses a BUCHI290 spray dryer operating within the following parameters:

| Process parameter | Set value |
|---|---|
| Inlet Temp | 65-95 °C |
| Q-Flow | 20~30mm |
| Outlet Temp | 40~70 °C |
| Pump | 15%~20% |
| Cooling Temp | -2 °C |
| Nozzle Cleaner | 2 |

The powder obtained through the spray drying described above may be further subjected to secondary drying, such as reduced-pressure drying, for example, at a temperature of 25-50 °C (e.g., 50 °C) and a reduced pressure of 0 Mpa to -0.1 Mpa (e.g., -0.1 Mpa) for a suitable period of time, such as 2-5 h, for example, 4 h.

### Example 1

The solubility (25 °C) of the active ingredient compound A in various solvents was determined using a standard solubility determination method well known in the art. The results are shown in Table 1 below:

**Table 1**

| Solvent | Solubility (mg/mL) | Solvent | Solubility (mg/mL) |
|---|---|---|---|
| Methanol | 4.3 | Methanol: dichloromethane (3:1) | 7.7 |
| Ethanol | 1.1 | Methanol: dichloromethane (2:1) | 14.3 |
| Acetonitrile | 1.2 | Methanol: dichloromethane (1:1) | 28.5 |
| N-methylpyridine | 11 | Methanol: dichloromethane (1:2) | 50 |
| Dichloromethane | 10 | Methanol: dichloromethane (1:3) | 83 |
| Tetrahydrofuran | 2 | Methanol: dichloromethane (1:4) | 83 |

| Toluene | 3.3 | Methanol: dichloromethane (1:5) | 83 |
|---|---|---|---|
| DMSO | 33 | | |
| DMF | 33 | | |

Based on the above experimental results, a mixed solvent of dichloromethane and methanol was used as a solvent system for preparing the solid dispersion in the present disclosure by comprehensively considering factors such as cost, dissolution efficiency, and toxicity.

### Example 2

12 mg of each type of carrier substance was weighed out and placed in a 2 mL liquid phase vial along with 8 mg of free base crystal of compound A, and then 1 mL of solvent dichloromethane:methanol (2:1) was added. The mixtures were stirred at 1500 rpm on a magnetic stirrer for 30 min until complete dissolution to formulate suspension solutions with a solid content of 20 mg/mL. 20 µL of each formula solution was pipetted and dropped onto a glass slide, and the drops were dispersed with a cover slip. The solution was dried at 50 °C and observed using polarized light microscopy (PLM). The corresponding detection of Eudragit L100-55 was performed using XRPD. The results are shown in Table 2:

**Table 2**

| **Carrier material** | **PLM/XRPD** |
|---|---|
| Hydroxypropyl methylcellulose acetate succinate-LG | No significant birefringent particles observed by PLM |
| Hydroxypropyl methylcellulose acetate succinate-MG | A very small number of birefringent particles observed by PLM |
| Hydroxypropyl methylcellulose acetate succinate-126G | A very small number of birefringent particles observed by PLM |
| Copovidone VA64 | A very small number of birefringent particles observed by PLM |
| Copovidone S630 | A very small number of birefringent particles observed by PLM |
| Povidone K29/32 | A very small number of birefringent particles observed by PLM |
| Hydroxypropyl cellulose EXF | A very small number of birefringent particles observed by PLM |
| Hydroxypropyl methylcellulose E4M | Not applicable due to excessively high viscosity |
| Polyethylene glycol 6000 | Significant insolubles/a large number of fine birefringent particles observed by PLM |
| Hydroxypropyl methylcellulose phthalate | A very small number of birefringent particles observed by PLM |
| Eudragit L100-55 | No significant crystal diffraction peaks detected by XRPD |

Table 2 shows that, among the tested carrier substances, polyethylene glycol and hydroxypropyl methylcellulose E4M were not significantly applicable, and other types of carriers were all able to completely convert the crystal form of compound A.

### Example 3

According to the solid dispersion preparation method described above, the following solid dispersions (ASDs) were prepared with different carriers and compound A by using dichloromethane:methanol (2:1) as the solvent system:

| ASD | Polymeric carrier | Drug loading ratio |
|---|---|---|
| ASD-1 | HPMCAS-LG | 1:3 |
| ASD-2 | Copovidone VA64 | 1:3 |
| ASD-3 | Hydroxypropyl cellulose EXF | 1:3 |

The following solid dispersions (ASDs) were prepared with different carriers and compound A by using dichloromethane:methanol (3:1) as the solvent system:

| ASD | Polymeric carrier in ASD | Drug loading ratio | Others |
|---|---|---|---|
| ASD-4 | Eudragit L100-55 | 1:2 | |
| ASD-5 | Eudragit L100-55 | 1:3 | |
| ASD-6 | Eudragit L100-55 | 1:4 | |
| ASD-7 | HPMCAS Eudragit L100-55 | API:HPMCAS=1:2 | With 10% Eudragit L100-55 added internally |
| ASD-8 | HPMCAS Eudragit L100-55 | API:HPMCAS=1:3 | With 10% Eudragit L100-55 added internally |
| ASD-9 | -- | 1:0 | |
| ASD-10 | HPMCAS-LG | 1:1 | |
| ASD-11 | HPMCAS-LG | 1:2 | |
| ASD-12 | HPMCAS-LG | 1:3 | |
| ASD-13 | HPMCAS-LG | 1:4 | |
| ASD-14 | HPMCAS-LG | 1:4 | With 5% HPMC-E4M added externally |
| ASD-15 | HPMCAS-LG | 1:4 | With 5% SDS added externally |
| ASD-16* | HPMCAS-LG | 1:4 | With 60 mg of HPMCAS-LG added externally |
| ASD-17 | HPMCAS-LG | 1:4 | With 5% Eudragit L100-55 added externally |
| ASD-18 | HPMCAS-LG | 1:2 | With 5% Eudragit L100-55 added externally |
| ASD-19 | HPMCAS-LG | 1:2 | With 10% Eudragit L100-55 added externally |
| ASD-20 | HPMCAS-LG | 1:2 | With 15% Eudragit L100-55 added externally |
| ASD-21 | HPMCAS-LG | 1:3 | With 5% Eudragit L100-55 added externally |
| ASD-22 | HPMCAS-LG | 1:3 | With 10% Eudragit L100-55 added externally |
| ASD-23 | HPMCAS-LG | 1:3 | With 15% Eudragit L100-55 added externally |
| ASD-24 | HPMCAS-LG | 1:4 | With 10% Eudragit L100-55 added externally |

| | | | |
|---|---|---|---|
| Note: "Added externally" means the substances were added to the solid dispersions already prepared with the carrier and compound A listed in the table; * indicates that the final ratio of compound A to HPMCAS-LG was 1:7. | | | |

The experimental results show that the solid dispersions of compound A prepared using different carriers and drug loading ratios all achieved a yield of no less than 75% and were all preparable.

### Example 4

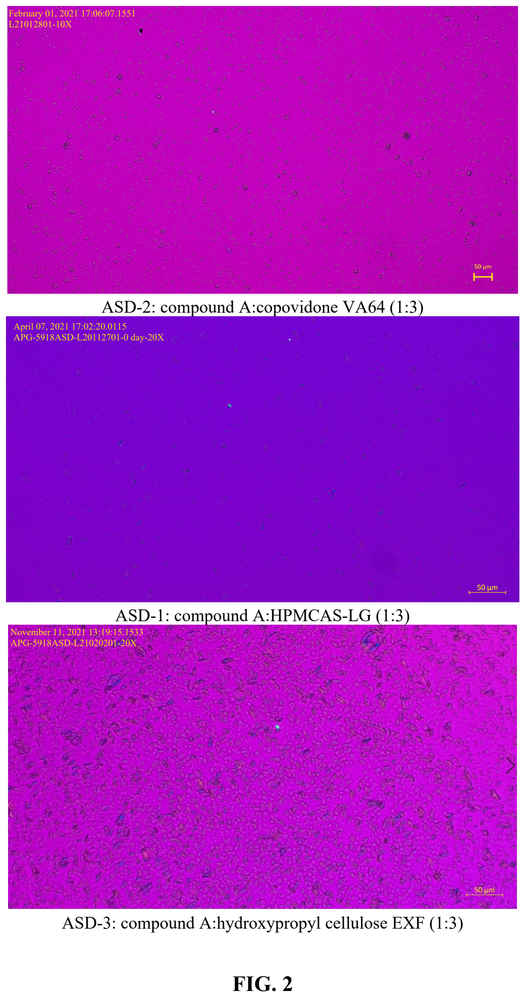

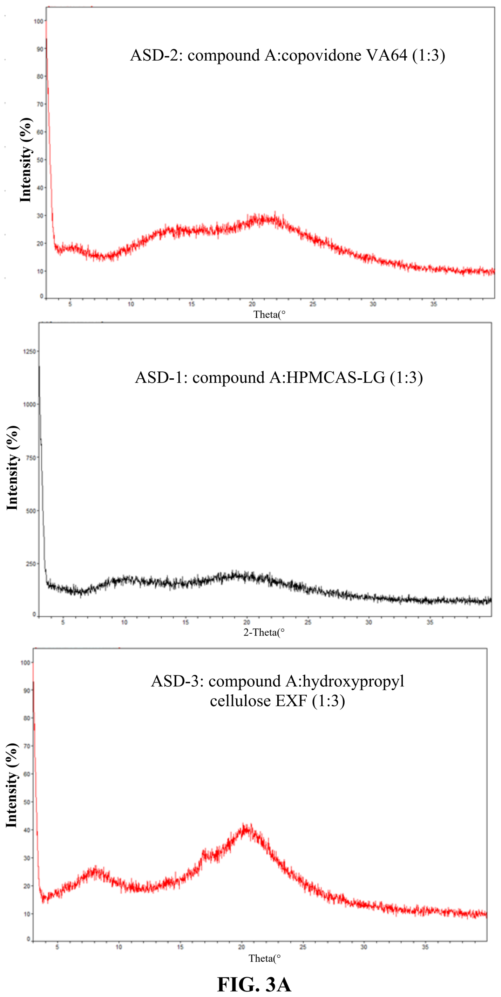

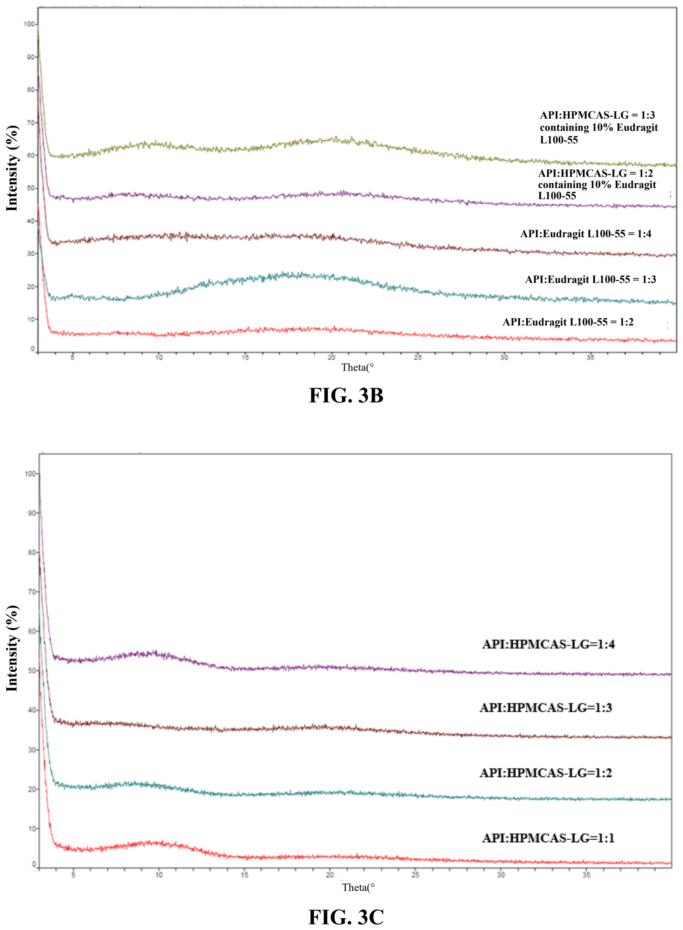

The crystal form conversion of compound A in the solid dispersions of the present disclosure was investigated using the X-ray powder diffraction (XRPD) and polarized light microscopy (PLM) determination methods described herein. The results are shown in Table 3 and FIGs. 2, 3A, 3B, and 3C:

**Table 3**

| | PLM | XRPD |
|---|---|---|
| ASD-1:API:HPMCAS-LG (1:3) | A very small number of birefringent particles | No crystal diffraction peak for compound A |
| ASD-2: API:copovidone VA64 (1:3) | A very small number of birefringent particles | No crystal diffraction peak for compound A |
| ASD-3: API:hydroxypropyl cellulose EXF (1:3) | A very small number of birefringent particles | No crystal diffraction peak for compound A |
| ASD-4:API:Eudragit L100-55 (1:2) | N/A | No crystal diffraction peak for compound A |
| ASD-5:API:Eudragit L100-55 (1:3) | N/A | No crystal diffraction peak for compound A |
| ASD-6:API:Eudragit L100-55 (1:4) | N/A | No crystal diffraction peak for compound A |
| ASD-7: API:HPMCAS-LG (1:2) containing 10% Eudragit L100-55 | N/A | No crystal diffraction peak for compound A |
| ASD-8: API:HPMCAS-LG (1:3) containing 10% Eudragit L100-55 | N/A | No crystal diffraction peak for compound A |
| ASD-10:API:HPMCAS-LG (1:1) | A very small number of birefringent particles | No crystal diffraction peak for compound A |
| ASD-11:API:HPMCAS-LG (1:2) | A very small number of birefringent particles | No crystal diffraction peak for compound A |
| ASD-12:API:HPMCAS-LG (1:3) | A very small number of birefringent particles | No crystal diffraction peak for compound A |
| ASD-13:API:HPMCAS-LG (1:4) | A very small number of birefringent particles | No crystal diffraction peak for compound A |

Table 3 shows that using HPMCAS-LG, copovidone VA64, hydroxypropyl cellulose EXF, and/or Eudragit L100-55 as carriers to prepare solid dispersions resulted in the complete crystal form conversion of compound A, with no significant crystal present.

The PLM detection results in Table 3 show that only a very small number of birefringent particles were observed in solid dispersions with different drug loading ratios. The XRPD results also indicate the absence of crystal diffraction peaks for compound A in all samples, demonstrating that complete crystal form conversion of compound A was achieved across all drug loading ratios.

The dynamic crystal form conversion of representative solid dispersions of the present disclosure was also investigated in an FeSSIF-V2 solution and/or an FaSSIF-V2 solution. Each solid dispersion was dispersed in the FeSSIF-V2 solution to formulate a suspension containing about 1 mg/mL of compound A, or in the FaSSIF-V2 solution to formulate a suspension containing about 2 mg/mL of compound A. After initial dispersion of each suspension on a magnetic stirrer, the suspensions were further dispersed at 1500 rpm at room temperature, and then the stirring was maintained at 1000 rpm. Samples of 1.5 mL were taken at fixed time points and observed using the polarized light microscopy (PLM) determination method described herein. The results are shown in Table 4:

**Table 4**

| ASD | PLM (FeSSIF-V2) (solution) | | | | |
|---|---|---|---|---|---|
| | 2h | 4h | 6h | 19h | 24h |
| ASD-4 | No significant birefringent particles | No significant birefringent particles | N/A | N/A | No significant birefringent particles |
| ASD-5 | No significant birefringent particles | No significant birefringent particles | N/A | N/A | No significant birefringent particles |
| ASD-6 | No significant birefringent particles | No significant birefringent particles | N/A | N/A | No significant birefringent particles |
| ASD-7 | No significant birefringent particles | No significant birefringent particles | N/A | N/A | No significant birefringent particles |
| ASD-8 | No significant birefringent particles | No significant birefringent particles | N/A | N/A | No significant birefringent particles |
| ASD-13 | No significant birefringent particles | No significant birefringent particles | N/A | A large number of birefringent particles | A large number of birefringent particles |
| ASD-14 | No significant birefringent particles | No significant birefringent particles | N/A | A large number of birefringent particles | N/A |
| ASD-15 | No significant birefringent particles | No significant birefringent particles | N/A | A small number of birefringent particles | N/A |
| ASD-16 | No significant birefringent particles | No significant birefringent particles | N/A | A large number of birefringent particles | N/A |
| ASD-17 | No significant birefringent particles | No significant birefringent particles | N/A | No significant birefringent particles | N/A |
| ASD-11 | No significant birefringent particles | No significant birefringent particles | A small number of birefringent particles | N/A | A large number of birefringent particles |
| ASD-18 | No significant birefringent particles | No significant birefringent particles | No significant birefringent particles | N/A | No significant birefringent particles |
| ASD-20 | No significant birefringent particles | No significant birefringent particles | No significant birefringent particles | N/A | No significant birefringent particles |
| ASD-12 | No significant birefringent particles | No significant birefringent particles | No significant birefringent particles | N/A | A large number of birefringent particles |
| ASD-21 | No significant birefringent particles | No significant birefringent particles | No significant birefringent particles | N/A | No significant birefringent particles |
| ASD-23 | No significant birefringent particles | No significant birefringent particles | No significant birefringent particles | N/A | No significant birefringent particles |

Table 4 shows that the solid dispersions (ASD-11, ASD-12, ASD-13) prepared using HPMCAS-LG (as the carrier) and compound A remained stable in the FeSSIF-V2 solution for about 6 h, with no significant birefringent particles observed by PLM, indicating no crystal form conversion. Prolonged stability in the FeSSIF-V2 solution could be achieved by additionally adding specific polymers to the solid dispersions. For example, the additional addition of HPMC-E4M, SDS, or HPMCAS-LG did not improve the stability in the solution after a long period of time, as a large number of birefringent particles were still observed at 19 h (ASD-14, ASD-15, ASD-16). However, the additional addition of Eudragit L100-55 to ASDs across various drug loading ratios enabled the solid dispersions to remain stable for up to 24 h in the FeSSIF-V2 solution, with no significant birefringent particles observed by PLM, indicating no crystal form conversion.

Table 4 also shows that the solid dispersions (ASD-4, ASD-5, ASD-6, ASD-7, and ASD-8) could also be prepared using Eudragit L100-55 or Eudragit L100-55 + HPMCAS-LG (as the carrier) and compound A, and this group of solid dispersions remained stable in the FeSSIF-V2 solution for up to 24 h, with no significant birefringent particles observed by PLM, indicating no crystal form conversion. Furthermore, this group of solid dispersions also remained stable in the FaSSIF-V2 solution for up to 24 h, with no significant birefringent particles observed by PLM at any time point, indicating no crystal form conversion.

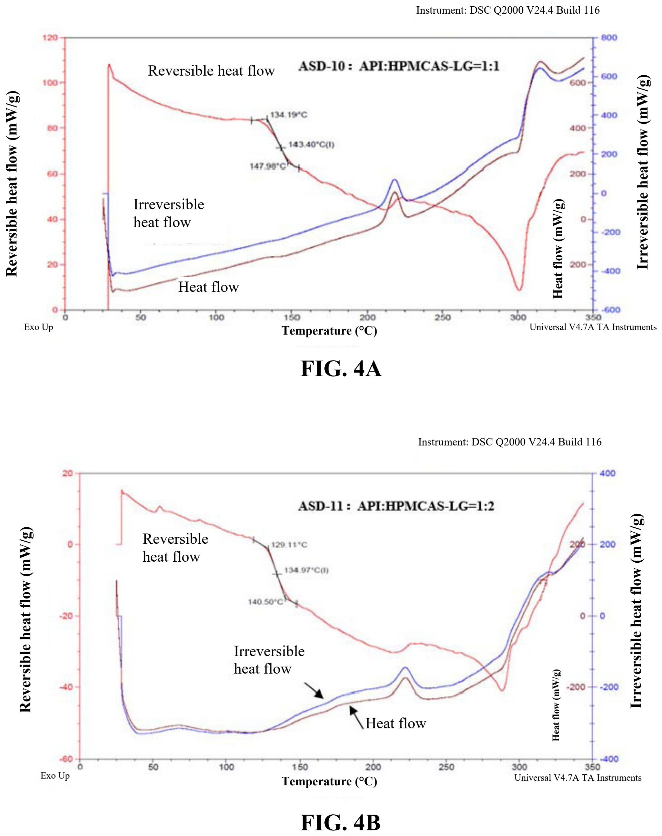

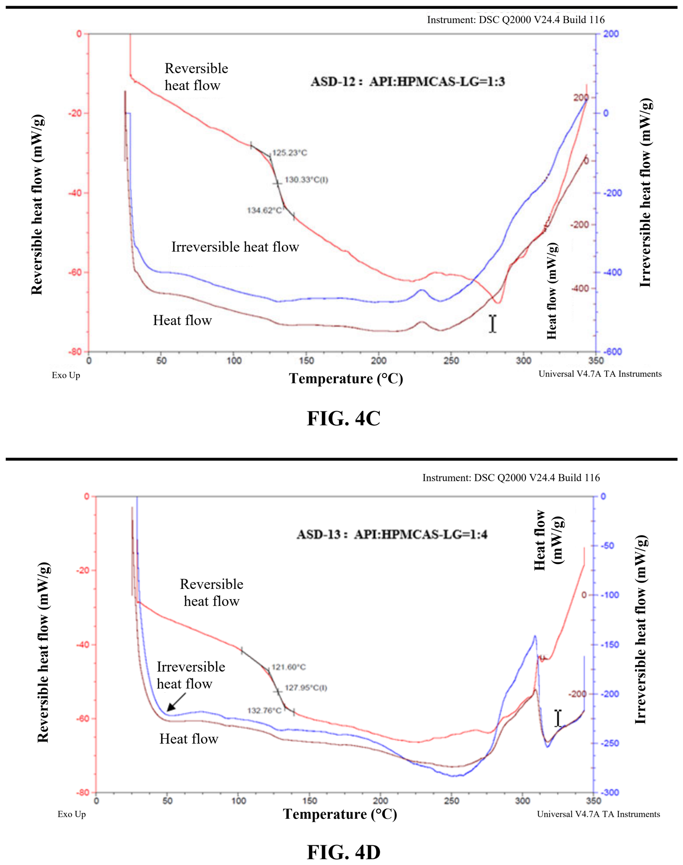

The crystal form conversion of compound A in solid dispersions of the present disclosure with different drug loading ratios (ASD-10, ASD-11, ASD-12, and ASD-13) was also investigated using the modulated differential scanning calorimetry (mDSC) determination method described herein. The results are shown in FIGs. 4A-4D. None of the samples showed a crystal peak for compound A. As the drug loading ratio decreased, the glass transition temperature decreased, and the intensity of the crystal form conversion peak at about 230 °C weakened.

### Example 5

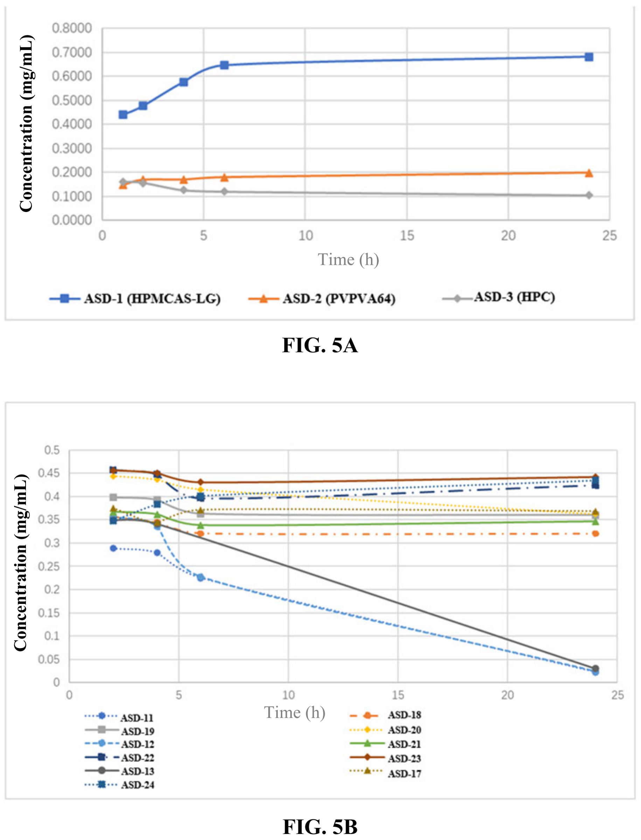

According to the general experimental methods described above, FaSSIF, FeSSIF, and SGF solutions were used to investigate the dynamic solubility of the solid dispersions of the present disclosure in various solutions. The solid dispersions were each dispersed in the FeSSIF-V2 solution to formulate suspensions containing about 1 mg/mL of compound A, or in the FaSSIF-V2 solution to formulate suspensions containing about 2 mg/mL of compound A. Each suspension was stirred for dispersion. At fixed time points, samples of 1-1.5 mL were taken and centrifuged, and the supernatant was collected and filtered through a membrane. The content of compound A was determined using the HPLC method described in the section of general experimental methods. The results are shown in Table 5 and FIGs. 5A and 5B.

**Table 5**

| Solid dispersion | Solubility (mg/mL) FaSSIF-V2 | | | | |
|---|---|---|---|---|---|
| | 1h | 2h | 4h | 6h | 24h |
| ASD-1:API:HPMCAS-LG (1:3) | 0.4396 | 0.4770 | 0.5763 | 0.6460 | 0.6816 |
| ASD-2: API:copovidone VA64 (1:3) | 0.1485 | 0.1701 | 0.1710 | 0.1804 | 0.1983 |
| ASD-3: API:hydroxypropyl cellulose EXF (1:3) | 0.1603 | 0.1559 | 0.1259 | 0.1203 | 0.1055 |
| ASD-4:API:Eudragit L100-55 (1:2) | N/A | 0.3848 | 0.4339 | N/A | 0.4503 |
| ASD-5:API:Eudragit L100-55 (1:3) | N/A | 0.2576 | 0.2604 | N/A | 0.2717 |
| ASD-6:API:Eudragit L100-55 (1:4) | N/A | 0.1956 | 0.1961 | N/A | 0.2165 |
| ASD-7: API:HPMCAS-LG (1:2) containing 10% Eudragit L100-55 | N/A | 0.7844 | 0.9731 | N/A | 1.0056 |
| ASD-8: API:HPMCAS-LG (1:3) containing 10% Eudragit L100-55 | N/A | 0.7435 | 0.8232 | N/A | 0.8260 |
| ASD-9: amorphous API | 0.0214 | 0.0226 | 0.0231 | 0.0234 | 0.0054 |
| ASD-10:API:HPMCAS-LG (1:1) | 0.5025 | 0.5917 | 0.7717 | 0.9200 | 1.2723 |
| ASD-11:API:HPMCAS-LG (1:2) | 0.9686 | 1.0845 | 1.0944 | 1.1703 | 1.2903 |
| ASD-12:API:HPMCAS-LG (1:3) | 0.9257 | 0.9139 | 1.0342 | 1.1202 | 1.2767 |
| ASD-13:API:HPMCAS-LG (1:4) | 0.8062 | 0.9731 | 1.0931 | 1.0468 | 1.2711 |
| | | | | | |

| | Solubility (mg/mL) FeSSIF-V2 | | | | |
|---|---|---|---|---|---|
| ASD-4:API:Eudragit L100-55 (1:2) | N/A | 0.3091 | 0.3146 | N/A | 0.3421 |
| ASD-5:API:Eudragit L100-55 (1:3) | N/A | 0.3367 | 0.3507 | N/A | 0.3837 |
| ASD-6:API:Eudragit L100-55 (1:4) | N/A | 0.3558 | 0.3568 | N/A | 0.3743 |
| ASD-7: API:HPMCAS-LG (1:2) containing 10% Eudragit L100-55 | N/A | 0.3487 | 0.3510 | N/A | 0.3513 |
| ASD-8: API:HPMCAS-LG (1:3) containing 10% Eudragit L100-55 | N/A | 0.4792 | 0.4760 | N/A | 0.4671 |
| ASD-11:API:HPMCAS-LG (1:2) | N/A | 0.2886 | 0.2791 | 0.2253 | 0.0227 |
| ASD-18: API:HPMCAS-LG (1:2) with 5% Eudragit L100-55 added externally | N/A | 0.3561 | 0.3446 | 0.3209 | 0.3198 |
| ASD-20: API:HPMCAS-LG (1:2) with 15% Eudragit L100-55 added externally | N/A | 0.4442 | 0.4364 | 0.4153 | 0.3624 |
| ASD-12:API:HPMCAS-LG (1:3) | N/A | 0.3566 | 0.3361 | 0.2275 | 0.0241 |
| ASD-21: API:HPMCAS-LG (1:3) with 5% Eudragit L100-55 added externally | N/A | 0.3672 | 0.3620 | 0.3388 | 0.3466 |
| ASD-23: API:HPMCAS-LG (1:3) with 15% Eudragit L100-55 added externally | N/A | 0.4560 | 0.4503 | 0.4308 | 0.4419 |
| ASD-13:API:HPMCAS-LG (1:4) | N/A | 0.3491 | 0.3414 | | 0.0300 |
| ASD-17: API:HPMCAS-LG (1:4) with 5% Eudragit L100-55 added externally | N/A | 0.3749 | 0.3449 | 0.3713 | 0.3686 |
| ASD-24: API:HPMCAS-LG (1:4) with 10% Eudragit L100-55 added externally | N/A | 0.3478 | 0.3837 | 0.4018 | 0.4350 |

| | | | | | |
|---|---|---|---|---|---|
| *: The API concentration of the FeSSIF-V2 suspension was 2 mg/mL. | | | | | |

Table 5 shows that, in the FaSSIF-V2 solution, the dissolved amounts of compound A solid dispersions with different carriers and drug loading ratios at various time points were far greater than that of the compound A starting material. The 24-h dissolved amounts were generally similar across the compound A solid dispersions with different drug loading ratios.

Table 5 also shows that, in the FaSSIF-V2 solution, the API solubility of solid dispersions prepared with HPMCAS and/or Eudragit L100-55, or with copovidone VA64, remained stable or increased over time. The API solubility of solid dispersions prepared with HPMCAS and having Eudragit L100-55 added internally showed a significant advantage over that prepared with copovidone VA64 or hydroxypropyl cellulose EXF.

Table 5 also shows that, in both FaSSIF-V2 and FeSSIF-V2 solutions, the solid dispersions containing Eudragit L100-55 (either added internally or externally) showed varying degrees of increase in API solubility compared to the solid dispersions with HPMCAS as the sole polymeric carrier.

Table 5 also shows that, in the FeSSIF-V2 solution, the dissolved amount increased as the drug loading amount decreased. The solid dispersions with HPMCAS as the sole polymeric carrier showed a significant decrease in dissolved amount at 24 h. However, the additional addition of Eudragit L100-55 to the solid dispersion enabled the dissolved amount to basically remain stable over time, and the dissolved amount of API increased as the ratio of added Eudragit L100-55 increased.

The comparison results in Table 5 also show that, under otherwise identical conditions, the dissolved amount of API in the FeSSIF-V2 solution was essentially the same whether Eudragit L100-55 was added internally or externally.

In addition, the solid dispersions of the present disclosure were formulated into suspensions containing about 2 mg/mL of compound A using an SGF solution, and the dynamic solubility was investigated. The results show that the API solubility of the solid dispersions with different drug loading ratios (e.g., ASDs 10-13) was less than 0.005 mg/mL, even less than 0.0001 mg/mL at each time point within a period of 1-24 h.

### Example 6

The solid dispersion ASD-12 (API:HPMCAS-LG = 1:3) prepared in Example 3 was left to stand under the conditions of high temperature (60 °C), high humidity (25 °C/RH90%±5%), and acceleration (40 °C±2 °C/RH75%±5%) for 0/5/10/30 days. Samples were taken, and the chemical stability of the ASD was evaluated by taking purity and related substances (maximum single impurity and total impurity) as investigation indices. The results are shown in Table 6.

**Table 6**

| | ASD-12 | | | | | | | | | |
|---|---|---|---|---|---|---|---|---|---|---|
| Condition | | 60 °C | | | 90%RH-25 °C | | | 75%RH-40 °C | | |
| Sampling time | 0d | 5d | 10d | 30d | 5d | 10d | 30d | 5d | 10d | 30d |
| Purity% | 99.670 | 99.645 | 99.686 | 99.043 | 99.715 | 99.654 | 99.488 | 99.625 | 99.694 | 99.181 |
| Maximum single impurity% | 0.104 | 0.102 | 0.104 | 0.119 | 0.099 | 0.103 | 0.104 | 0.098 | 0.101 | 0.082 |
| Total impurity% | 0.27 | 0.25 | 0.23 | 0.72 | 0.22 | 0.22 | 0.37 | 0.24 | 0.21 | 0.60 |

Table 6 shows that: the ASD exhibited no significant change in appearance and no significant increase in related substances under the conditions of high temperature, high humidity, and acceleration, indicating that the chemical stability of the ASD was good.

In addition, the solid-state stability (by mDSC, XRPD, and PLM) of the samples of the solid dispersion ASD-1 taken at the time points described above was also investigated. The results show that, on day 30, no significant crystal diffraction was detected in the XRPD patterns of all samples, and PLM examination revealed no significant birefringent particles. Furthermore, the mDSC patterns of samples under all conditions showed no significant change compared to day 0.

Furthermore, the content, impurities (single and total impurities), and solid-state stability (by mDSC, XRPD, and PLM) of solid dispersion ASD-1 were also investigated under the following conditions: refrigeration (2-8 °C), grinding for 5 min (room temperature), grinding for 5 min followed by refrigeration (2-8 °C), grinding for 15 min (room temperature), grinding for 15 min followed by refrigeration (2-8 °C), tableting (room temperature), tableting followed by refrigeration (2-8 °C), tableting with PH102 at room temperature (solid dispersion:PH102 = 1:5), and tableting with PH102 at 2-8 °C (solid dispersion:PH102 = 1:5). Sampling was performed on days 0, 5, 10, and 30. The results show that no significant changes in content and impurities were observed in the samples under each test condition, no significant crystal diffraction was detected in the XRPD patterns of all samples, and PLM examination revealed no significant birefringent particles. Furthermore, the mDSC patterns of samples under all conditions showed no significant change compared to day 0.

The above results show that the solid dispersions of the present disclosure have satisfactory stability properties and show excellent chemical stability and solid-state stability under all the test conditions, so that the solid dispersions have the potential to be developed into practical and convenient formulation products.

### Example 7

This experiment investigated the pharmacokinetic properties of the solid dispersion of the present disclosure (ASD-22, filled in capsule shells) in beagle dogs, using a 2.5 mg/mL solution of compound A in 0.5% aqueous SDS solution (formulated within 2 h before use) as the control.

Specifically, 15 beagle dogs (male, body weight of about 8-11 kg) were randomly divided into 5 groups, with 3 dogs in each group. After one night of fasting, two groups were orally given the test sample at doses of 2.5 mg/kg and 10 mg/kg, respectively, and the beagle dogs in another group were orally given the test sample at a dose of 10 mg/kg 0.5 h after feeding. The beagle dogs in the remaining two groups, after one night of fasting, were orally given a compound A solution at 2.5 mg/kg and 50 mg/kg, respectively, as controls. 0.5 mL of blood was separately collected from the cephalic vein before administration (0 h) and 0.083 h, 0.25 h, 0.5 h, 1 h, 2 h, 4 h, 6 h, 8 h, and 24 h after administration. After collection, the blood samples were immediately placed into heparinized EP tubes and centrifuged at 3000 g (RCF: relative centrifugal force) for 5 min to separate the plasma, which was then cryopreserved in a refrigerator at - 70 °C for later analysis.

During the determination, the plasma was thawed, and 20 µL of the thawed plasma was then precisely pipetted into a centrifuge tube with a stopper, and 200 µL of an internal standard solution (aqueous solution of 60 ng/mL diclofenac in 70% methanol) was precisely added. The mixture was vortexed for 10 min for uniform mixing and centrifuged at 5800 rpm for 10 min. The supernatant was taken for LC-MS/MS analysis (Waters ACQUITY UPLC BEH C18 2.1 × 50 mm; mobile phase A: a 0.025% formic acid (1 mM aqueous ammonium acetate solution) solution, mobile phase B: a 0.025% phosphoric acid (solution of 1 mM ammonium acetate in methanol) solution; gradient: 10% B for 0.2 min, increased to 95% B from 0.2 min to 1 min, held for 0.5 min, decreased to 10% B from 1.50 min to 1.51 min, held until 2 min; flow rate: 0.60 mL/min; autosampler controlled temperature/column temperature: 5 °C/60 °C; injection volume: 4 µL).

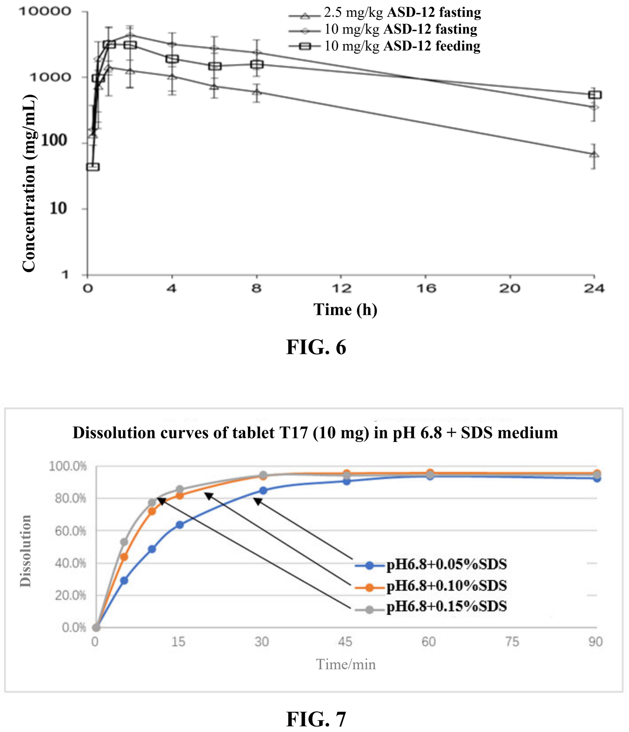

The main pharmacokinetic parameters are shown in Table 7, and the plasma drug concentration-time curves are shown in FIG. 6, wherein AUC values were calculated using WinNonLin software.

**Table 7**

| Pharmacokinetic parameter | Solid dispersion (capsule) | | | Solution | |
|---|---|---|---|---|---|
| | 2.5 mg/kg Fasting | 10 mg/kg Fasting | 10 mg/kg Feeding | 2.5 mg/kg Fasting | 50 mg/kg Fasting |
| Tₘₐₓ (hr) | 1.33 | 1.33 | 3.67 | 1.7 | 3.0 |
| Cₘₐₓ (ng/mL) | 1500 | 5130 | 3790 | 3164 | 8770 |
| AUCₗₐₛₜ (hr*ng/mL) | 12767 | 45973 | 32934 | 15326 | 156090 |
| AUC_{INF} (hr*ng/mL) | 13294 | 49022 | 43492 | 15599 | 156406 |
| T_{1/2} (hr) | 5.26 | 6.09 | 9.21 | 4.82 | 8.27 |

As shown in Table 7 and FIG. 6, the exposures of the solid dispersion of the present application in beagle dogs were comparable to those of the solution dosage form at the same dose under the same conditions, and no significant food effect was observed. This indicates that compound A, which is almost insoluble in water, was successfully prepared into a solid form with bioavailability comparable to that of a solution. This overcomes the common drawback of low bioavailability for poorly soluble drugs and expands the application scenarios of the drugs.

### Example 8

The solid dispersion of the present disclosure (ASD-12) was uniformly mixed with the listed excipients according to the formulas shown in Table 8 below, and the mixtures were sieved and subjected to dry granulation. The prepared granules were uniformly mixed with magnesium stearate, and the obtained final mixed granules were compressed into tablets.

**Table 8**

| Ingredient/Formula No. | | T1 | T2 | T3 | T4 | T5 | T6 | T7 | T8 |
|---|---|---|---|---|---|---|---|---|---|
| ASD-12 | | 50% | 70.2% | 50.0% | 50.0% | 59.7% | 50% | 50% | 50% |
| Eudragit L100-55 | | 5.6% | 7.8% | 5.6% | 5.6% | 6.6% | 5.6% | 5.6% | 5.6% |
| Lactose | | 19.2% | 8.0% | 38.4% | / | / | 19.2% | 19.2% | |
| MCC KG802 | | 19.2% | 8.0% | | 38.4% | 27.7% | | | 19.2% |
| CaHPO₄ | | | | | | | 19.2% | | 19.2% |
| Ca₃(PO₄)₂ | | | | | | | | 19.2% | |
| CCNa | | 5.0% | 5.0% | 5.0% | 5.0% | 5.0% | 5.0% | 5.0% | 5.0% |
| MS (added externally) | | 1.0% | 1.0% | 1.0% | 1.0% | 1.0% | 1.0% | 1.0% | 1.0% |
| Tablet weight (mg) | | 400 | 285 | 400 | 400 | 335 | 400 | 400 | 400 |
| Friability (4 min) | | 0.08% Wear | 0.11% Slight edge defect | 0.10% Intact | 0.26% Slight edge defect | 0.09% Slight edge defect | 0.26% Intact | 0.25% Wear | 0.19% Intact |
| Friability (8 min) | | 0.15% Wear | 0.12% Slight edge defect | 0.16% Slight wear | 0.36% Slight edge defect | 0.22% Slight edge defect | 0.26% Slight wear | 0.34% Wear | 0.14% Slight wear |
| Dry granulated granules | | Relatively hard | Sticky to rollers | Moderate | Relatively hard | Slightly hard | Moderate | Moderate | Moderate |
| Hardness (N) | | 163.3 | 113.3 | 146.3 | 178.2 | 133.1 | 135.4 | 132.0 | 130.2 |
| Disintegration time | | 5min35s | 28min15s | 6min10s | 5min18s | 7min30s | 3min28s | 2min12s | 2min22s |
| Dissolution (pH 6.8 + 0.1% SDS) | 5min | 6.7% | 5.9% | 6.7% | 11.6% | 6.7% | 5.8% | 18.3% | 12.1% |
| | 10min | 19.0% | 7.3% | 17.5% | 31.9% | 20.1% | 16.8% | 36.0% | 21.9% |
| | 15min | 34.7% | 8.8% | 32.2% | 48.3% | 34.6% | 25.2% | 50.3% | 30.0% |
| | 30min | 70.4% | 15.1% | 66.5% | 79.7% | 66.8% | 43.6% | 73.9% | 51.2% |
| | 45min | 81.9% | 23.4% | 81.2% | 89.5% | 81.4% | 56.9% | 78.5% | 61.4% |
| | 60min | 87.9% | 39.0% | 87.5% | 92.4% | 90.0% | 63.4% | 80.7% | 70.7% |

Table 8 shows that the tablets of formulas T1, T3, T4, and T5 exhibited relatively good dissolution characteristics, achieving no less than 80% dissolution within 45 min. In particular, tablets prepared using microcrystalline cellulose as the sole filler demonstrated the best overall performance, with good compressibility, an acceptable disintegration time limit, and the most complete dissolution.

The solid dispersion of the present disclosure (ASD-12) was also uniformly mixed with the listed excipients according to the formulas shown in Table 9 below, and the mixtures were sieved and subjected to dry granulation. The prepared granules were uniformly mixed with magnesium stearate, and the obtained final mixed granules were compressed into tablets.

**Table 9**

| Ingredient/Form ula No. | T9 | T10 | T11 | T12 | T13 | T14 | T15 | T16 |
|---|---|---|---|---|---|---|---|---|
| ASD-12 | 60.0% | 60.0% | 60.0% | 60.0% | 60.0% | 60.0% | 60.0% | 60.0% |
| Eudragit L100-55 | 6.7% | 6.7% | 6.7% | 6.7% | 6.7% | 6.7% | 6.7% | 6.7% |
| MCC KG802 | 28.3% | 20.8% | 25.8% | 23.8% | 20.8% | 23.8% | 24.3% | 24.55% |
| HPMC | | | | 2.0% | 5.0% | 2.0% | 2.0% | 2.0% |
| CCNa | 2.5% | 10.0% | 5.0% | 5.0% | 5.0% | 5.0% | 5.0% | 5.0% |
| SiO₂ | 1.0% | 1.0% | 1.0% | 1.0% | 1.0% | 1.0% | 1.0% | 1.0% |
| MS (added internally) | 1.0% | 1.0% | 1.0% | 1.0% | 1.0% | 1.0% | 0.5% | 0.5% |
| MS (added externally) | 0.5% | 0.5% | 0.5% | 0.5% | 0.5% | 0.5% | 0.5% | 0.25% |
| Tablet weight (mg) | 333 | 333 | 1333 | 1333 | 1333 | 1333 | 1333 | 1333 |
| Friability (4 min) | 0.12% Slight edge defect | 0.05% Slight edge defect | 0.14% Edge defect | 0.14% Slight edge defect | 0.08% Slight edge defect | 0.04% Slight edge defect | 0.15% Slight edge defect | 0.07% Slight edge defect |
| Friability (8 min) | 0.20% Slight edge defect | 0.11% Slight edge defect | 0.30% Edge defect | 0.16% Slight edge defect | 0.09% Slight edge defect | 0.09% Slight edge defect | 0.18% Slight edge defect | 0.09% Slight edge defect |
| Dry granulated granules | Moderate hardness | Moderate hardness | Moderate hardness | Moderate hardness* | Moderate hardness* | Moderate hardness | Moderate hardness | Moderate hardness |
| Tablet thickness (mm) | 4.96 | 4.98 | 7.74 | 7.73 | 7.76 | 8.04 | 7.79 | 8.09 |
| Hardness (N) | 137.6 | 133.0 | - | - | 387.0 | 288.3 | 324.8 | 300 |
| Disintegration time | 8min36s | 2min23s | 5min48s | 8min28s | - | - | 6min21s | - |

| | | | | | | | | |
|---|---|---|---|---|---|---|---|---|
| *: Dry granulation pressure was 5-6 KPa; all others were 6-8 KPa. | | | | | | | | |

The tablets prepared according to the above formulas all had acceptable off-white (occasionally speckled) appearance.

Table 9 shows that the disintegrant content was proportional to the disintegration time. About 1-10 wt% of the disintegrant croscarmellose sodium enabled rapid disintegration of the formulation within a disintegration time of 1-10 min, wherein about 2-8 wt%, particularly about 3-7 wt% of the disintegrant resulted in the most moderate disintegration time. The dry granulated granules of each formula had moderate hardness, with friability (4 min) of about 0.05-0.15% and friability (8 min) of about 0.1-0.3%.

Table 9 also shows that the tested content range of the binder enabled the hardness of the dry granulated granules to be moderate, wherein about 1-3 wt% of the binder was preferred, and about 1.5-2.5 wt% of the binder was most preferred. The compressed tablets had good formulation properties, such as friability of less than 0.2 wt% and a moderate disintegration time.

The formulation experimental process shows that adding the above-described formula amount of magnesium stearate to the initial mixed powder for dry granulation and performing uniform mixing prior to dry granulation helped mitigate adhesion. Adding the formula amount of magnesium stearate after dry granulation and performing tableting after uniform mixing helped mitigate sticking.

In addition, formula T15 in the above table was adopted, and the pressure of dry granulation was changed from 6-8 KPa to 4-6 KPa. It was observed that the granulated substances basically did not adhere to the roller, had good flowability after granulation, and showed no sticking during tableting. The tablet surface was flat and smooth, and the friability was qualified (all less than 0.1%). The disintegration time limit was less than 10 min (a disintegration time of 6 min and 8 s for a 50 mg tablet, a disintegration time of 3 min for a 200 mg tablet). The dissolution (pH 6.8 + 0.1% SDS) at 45 min reached 89.1% (50 mg tablet) and 90.7% (200 mg tablet), and the dissolution (pH 6.8 + 0.1% SDS) at 60 min reached 93.9% (50 mg tablet) and 91.7% (200 mg tablet).

### Example 9

Based on the investigation results from Example 8, the solid dispersions of the present disclosure, along with a stabilizer, a filler, a binder, a disintegrant, a glidant, a lubricant, and a film coating premix, were formulated into 10 mg, 50 mg, and 200 mg tablets, respectively, and the properties of the tablets were investigated.

Specifically, the formula amounts of solid dispersion ASD-12, a methacrylic acid-ethyl acrylate copolymer (e.g., Eudragit L100-55), microcrystalline cellulose, hydroxypropyl methylcellulose, croscarmellose sodium, colloidal silica, film coating premix, and magnesium stearate were weighed out according to Table 10 below and sieved (50-60 meshes). ASD-12, the methacrylic acid-ethyl acrylate copolymer, microcrystalline cellulose, hydroxypropyl methylcellulose, croscarmellose sodium, and colloidal silica were mixed in a mixer and then sieved, and this step could be repeated once (premix). Then, internally added magnesium stearate (about 0.5% w/w) was added, and the mixture was uniformly mixed. The premix to which magnesium stearate was added was subjected to dry granulation in a dry granulator, with the pressure controlled at 4-6 KPa. The prepared granules were uniformly mixed with the remaining magnesium stearate (about 0.5% w/w), and the obtained final mixed granules were compressed into tablets. The appearance, friability, hardness, dissolution properties, and stability of the obtained tablets were investigated.

**Table 10**

| **Ingredient** | **% (w/w)** | **Content per tablet (mg)** | | | | | |
|---|---|---|---|---|---|---|---|
| | | **T17 (10 mg)** | | **T18 (50 mg)** | | **T19 (200 mg)** | |
| ASD-12 | 58.25 | 40 | | 200 | | 800 | |
| Methacrylic acid-ethyl acrylate copolymer | 6.50 | 4.5 | | 22.3 | | 89.2 | |
| Microcrystalline cellulose | 23.57 | 16.2 | | 80.9 | | 323.6 | |
| Hydroxypropyl methylcellulose | 1.95 | 1.3 | | 6.7 | | 26.8 | |
| Croscarmellose sodium | 4.86 | 3.3 | | 16.7 | | 66.8 | |
| Colloidal silica | 0.96 | 0.7 | | 3.3 | | 13.2 | |
| Magnesium stearate | 1.00 | 0.7 | | 1.7 | | 6.8 | |
| Film coating premix | 2.91 | 2.0 | | 10.0 | | 40.0 | |
| Dichloromethane:methanol 3:1 (v/v) | | | | | | | |
| Purified water | | Used and eventually removed in the coating process | | | | | |
| Total | 100.00 | 68.7 | | 343.3 | | 1373.2 | |
| Actual tablet weight | | 67.2 | 66.7 | 338.2 | 334.8 | 1313.0 | 1324.7 |
| Hardness (N) | | *55.1* | 70.4 | 120.7 | 161.8 | 338.5 | 391.9 |
| Friability% (4 min) | | 0 Intact | 0 Intact | 0.0 Intact | 0.0 Intact | 0.06 Intact | 0.0 Intact |
| Friability% (8 min) | | 0 Intact | 0 Intact | 0.1 Intact | 0.0 Intact | 0.07 **Intact** | 0.0 Intact |
| Empty coating rotation | | No edge defects, no pitted surfaces | | | | | |
| Dissolution% pH 6.8 + 0.15% SDS | 5min | 83.8 | N/A | (65.3) | (56.6) | 53.1 | 48.4 |
| | 10min | 95.3 | N/A | (82.4) | (76.8) | 70.8 | 72.8 |
| | 15min | 96.3 | N/A | (88.2) | (84.2) | 78.7 | 80.7 |
| | 30min | 97.3 | N/A | (91.1) | (89.7) | 84.4 | 86.3 |
| | 45min | 98.5 | N/A | (92.9) | (91.3) | 86.4 | 89.1 |
| | 60min | 98.5 | N/A | (93.0) | (91.7) | 87.5 | 88.9 |

| | | | | | | | |
|---|---|---|---|---|---|---|---|
| Note: The dissolution data in parentheses in this table were obtained by testing in pH 6.8 + 0.1% SDS. | | | | | | | |

Table 10 shows that the poorly soluble compound A was prepared into a solid dispersion according to the present disclosure, the solid dispersion was further prepared into an oral solid formulation according to the present disclosure, and the formulation exhibited excellent dissolution performance (FIG. 7) and demonstrated good formulation properties. It is anticipated to provide an anti-cancer drug with enhanced bioavailability and thus therapeutic efficacy at lower doses, thereby satisfying medical needs in the field of cancer treatment.

Furthermore, the stability of the aforementioned 10 mg, 50 mg, and 200 mg tablets packaged in common packaging materials (e.g., 45 mL or 100 mL pharmaceutical high-density polyethylene bottles, 33 mm or 38 mm pharmaceutical polyethylene/polypropylene child-resistant combination caps) was investigated under conditions of 40 °C±2 °C/RH75%±5% and 30 °C±2 °C/RH65%±5%. The test samples were examined for appearance, water content, related substances, content, and dissolution. The results show no significant changes, indicating that the prepared tablet formulations possess good stability and are compatible with the packaging materials. The results are shown in Table 11:

**Table 11**

| **Investigation conditions: 40±2 °C/75 RH±5%RH** | | | | |
|---|---|---|---|---|
| | **T18 (50 mg tablet)** | | | |
| **Detection item/time** | **0 months** | **1 month** | **3 months** | **6 months** |
| Water content | 1.1% | 1.1% | 1.1% | 1.1% |
| Dissolution | 101% | 99% | 102% | 99% |
| Related substance | 0.1% | 0.1% | 0.1% | 0.2% |
| Content | 99.6% | 99.2% | 98.7% | 97.6% |

| | **T17 (10 mg tablet)** | | | |
|---|---|---|---|---|
| Water content | 3.9% | 2.9% | 3.1% | |
| Dissolution | 99% | 100% | 99% | |
| Related substance | 0.1% | 0.1% | 0.1% | |
| Content | 98.1% | 98.4% | 98.5% | |

| | **T19 (200 mg tablet)** | | | |
|---|---|---|---|---|
| Water content | 1.1% | 1.1% | 1.1% | 1.1% |
| Dissolution | 98% | 98% | 99% | 95% |
| Related substance | 0.1% | 0.1% | 0.1% | 0.1% |
| Content | 97.1% | 96.0% | 96.2% | 94.1% |

| **Investigation conditions: 30±2 °C/65 RH±5%RH** | | | | |
|---|---|---|---|---|
| | **T18 (50 mg tablet)** | | | |
| **Detection item/time** | **0 months** | **1 month** | **3 months** | **6 months** |
| Water content | 1.1% | 1.1% | 1.1% | 1.7% |
| Dissolution | 101% | 97% | 100% | 100% |
| Related substance | 0.1% | 0.1% | 0.1% | 0.1% |
| Content | 99.6% | 99.2% | 96.8% | 99.7% |

| | **T17 (10 mg tablet)** | | | |
|---|---|---|---|---|
| Water content | 3.9% | | 2.8% | |
| Dissolution | 99% | | 99% | |
| Related substance | 0.1% | | 0.1% | |
| Content | 98.1% | | 99.1% | |

| | **T19 (200 mg tablet)** | | | |
|---|---|---|---|---|
| Water content | 1.1% | 1.1% | 1.1% | 1.1% |
| Dissolution | 98% | 88% | 93% | 96% |
| Related substance | 0.1% | 0.1% | 0.1% | 0.1% |
| Content | 97.1% | 95.8% | 94.7% | 96.9% |

The dissolution value represents the experimental result at 45 min in the phosphate buffer (pH 6.8) + 0.15% sodium dodecyl sulfate medium. The "related substance" value is the sum of all impurities in the HPLC pattern under the HPLC detection conditions described in the section of general experimental methods. "Water content" is the result determined by the Karl Fischer method.

The specific embodiments provided herein are merely illustrative of the present disclosure and do not constitute limitations on the scope defined by the claims. On the basis of the content disclosed in the present application, it will be apparent to those skilled in the art that equivalent variants of the technical solutions of the present application are also encompassed within the scope of the present application.

## Claims

1. A solid dispersion, comprising an EED inhibitor or a pharmaceutically acceptable salt or solvate thereof, and a polymeric carrier, wherein the EED inhibitor is a compound of the following formula: 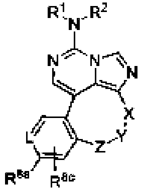 wherein:
R¹ is aralkyl;
R² is selected from H and C₁-C₄ alkyl;
X is selected from -C(R^{5a})(R^{5b})-, -C(=O)-, and -S(=O)₂-; R^{5a} and R^{5b} are independently selected from H and C₁-C₄ alkyl;
Y is selected from -C(R^{6a})(R^{6b})-, -S-, -O-, and -N(R⁷)-;
Z is -C(R^{6c})(R^{6d})ₘ-;
R^{6a} and R^{6b} are independently selected from H and C₁-C₄ alkyl;
R^{6c} and R^{6d} are each independently selected from H and C₁-C₄ alkyl; m is 0, 1, or 2;
R⁷ is selected from C₁-C₆ alkyl, C₁-C₆ haloalkyl, and optionally substituted C₃-C₈ cycloalkyl;
L is selected from -C(R^{8b})= and -N=;
R^{8a} is selected from -CF₃, -CH₃, -CHF₂, -CD₃, and cyclopropyl; and
R^{8b} and R^{8c} are hydrogen;
or a pharmaceutically acceptable salt or solvate thereof.

2. The solid dispersion according to claim 1, wherein the EED inhibitor is compound A: 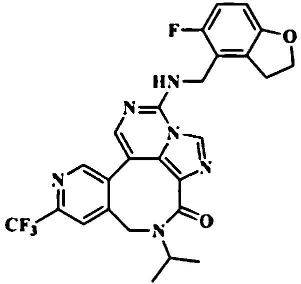 or a pharmaceutically acceptable salt or solvate thereof.

3. The solid dispersion according to claim 1 or 2, wherein the polymeric carrier is selected from a cellulose ether-ester, preferably hydroxypropyl methylcellulose acetate succinate.

4. The solid dispersion according to claim 1 or 2, wherein the polymeric carrier is selected from a polyacrylic acid resin, preferably a methacrylic acid-ethyl acrylate (1:1) copolymer, and preferably Eudragit L100-55.

5. The solid dispersion according to claim 1 or 2, wherein the polymeric carrier is a mixture of a cellulose ether-ester and a polyacrylic acid resin, preferably a mixture of hydroxypropyl methylcellulose acetate succinate and a copolymer of methacrylic acid and ethyl acrylate (1:1), and more preferably a mixture of hydroxypropyl methylcellulose acetate succinate and Eudragit L100-55.

6. The solid dispersion according to any one of claims 1 to 5, wherein the EED inhibitor or the pharmaceutically acceptable salt or solvate thereof and the polymeric carrier are in a weight ratio of about 1:1 to 1:5.

7. An oral solid formulation, comprising the solid dispersion according to any one of claims 1-6 and one or more pharmaceutically acceptable excipients.

8. The oral solid formulation according to claim 7, wherein the oral solid formulation is a tablet, and comprises the solid dispersion according to any one of claims 1-6 and one or more of a filler, a binder, a disintegrant, a lubricant, a glidant, a stabilizer, and a coating agent.

9. The oral solid formulation according to claim 7 or 8, wherein the pharmaceutically acceptable excipient is a filler, a binder, a disintegrant, a lubricant, a glidant, and a stabilizer.

10. The oral solid formulation according to any one of claims 7 to 9, wherein the EED inhibitor has a content of about 10-30 wt%, preferably about 10-20 wt%.

11. The oral solid formulation according to any one of claims 7 to 9, wherein the solid dispersion accounts for about 30-80%, preferably about 50-70% of the weight of the oral formulation.

12. The oral solid formulation according to any one of claims 7 to 9, comprising
1) about 30-80 wt% said solid dispersion comprising about 10-200 mg of the EED inhibitor and the polymeric carrier;
2) about 20-40 wt% filler;
3) about 1-3 wt% binder;
4) about 2-8 wt% disintegrant;
5) about 0.5-2 wt% lubricant;
6) about 2-8 wt% stabilizer; and
7) about 0.5-2 wt% glidant.

13. The oral solid formulation according to any one of claims 7 to 9, comprising
1) about 50-70 wt% said solid dispersion comprising about 10-200 mg of the EED inhibitor and the polymeric carrier;
2) about 20-30 wt% filler;
3) about 1.5-2.5 wt% binder;
4) about 3-7 wt% disintegrant;
5) about 0.5-1.5 wt% lubricant;
6) about 4-8 wt% stabilizer; and
7) about 0.5-1.5 wt% glidant.

14. The oral solid formulation according to any one of claims 8 to 13, wherein the EED inhibitor is compound A or a pharmaceutically acceptable salt or solvate thereof, and the polymeric carrier in the solid dispersion is selected from hydroxypropyl methylcellulose acetate succinate, a methacrylic acid-ethyl acrylate (1:1) copolymer, and a mixture thereof.

15. The oral solid formulation according to any one of claims 8 to 14, wherein the stabilizer is a methacrylic acid-ethyl acrylate (1:1) copolymer, preferably Eudragit L100-55.

16. The oral solid formulation according to any one of claims 8 to 15, wherein the filler is microcrystalline cellulose, the binder is hydroxypropyl methylcellulose, the disintegrant is croscarmellose sodium, the lubricant is magnesium stearate, and the glidant is colloidal silica.

17. Use of the solid dispersion according to any one of claims 1 to 6 or the oral solid formulation according to any one of claims 7-16 in treating or preventing a disease where inhibition of EED provides a benefit.

18. Use of the solid dispersion according to any one of claims 1 to 6 or the oral solid formulation according to any one of claims 7-16 in preparing a medicament for treating or preventing a disease where inhibition of EED provides a benefit.

19. A method for treating or preventing a disease where inhibition of EED provides a benefit in a patient in need, comprising administering a therapeutically effective amount of the solid dispersion according to any one of claims 1 to 6 or the oral solid formulation according to any one of claims 7-16 to the patient.

20. The use according to claim 17 or 18 or the method according to claim 19, wherein the disease where inhibition of EED provides a benefit is cancer selected from acute monocytic leukemia, acute myeloid leukemia, chronic myeloid leukemia, chronic lymphocytic leukemia, mixed lineage leukemia, NUT-midline carcinoma, multiple myeloma, small cell lung cancer, non-small cell lung cancer, neuroblastoma, Burkitt lymphoma, cervical cancer, esophageal cancer, ovarian cancer, colorectal cancer, prostate cancer, breast cancer, bladder cancer, ovarian cancer, glioma, sarcoma, esophageal squamous cell carcinoma, and papillary thyroid carcinoma.
